(19) Europäisches Patentamt
European Patent Office
Office européen des brevets

(11) **EP 4 323 101 B1**

(12) **EUROPÄISCHE PATENTSCHRIFT**

(45) Veröffentlichungstag und Bekanntmachung des Hinweises auf die Patenterteilung:
**06.11.2024 Patentblatt 2024/45**

(21) Anmeldenummer: **22720698.4**

(22) Anmeldetag: **05.04.2022**

(51) Internationale Patentklassifikation (IPC):
**B01J 19/00** (2006.01)

(52) Gemeinsame Patentklassifikation (CPC):
**B01J 19/0013;** B01J 2208/00265;
B01J 2219/00103; B01J 2219/00105;
B01J 2219/002; B01J 2219/0022; B01J 2219/00238

(86) Internationale Anmeldenummer:
**PCT/EP2022/058969**

(87) Internationale Veröffentlichungsnummer:
**WO 2022/218757 (20.10.2022 Gazette 2022/42)**

(54) **VERFAHREN ZUR REGELUNG DER TEMPERATUR IN EINEM VERFAHRENSTECHNISCHEN APPARAT**

METHOD FOR CONTROLLING THE TEMPERATURE IN A PROCESSING APPARATUS

PROCÉDÉ DE RÉGULATION DE LA TEMPÉRATURE DANS UN APPAREIL DE PROCESSUS TECHNIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2021 EP 21168533**

(43) Veröffentlichungstag der Anmeldung:
**21.02.2024 Patentblatt 2024/08**

(73) Patentinhaber: **BASF SE**
**67056 Ludwigshafen am Rhein (DE)**

(72) Erfinder:
- **HUETTEN, Frank**
  **67056 Ludwigshafen (DE)**
- **WOLFF, Florian**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**WO-A1-2011/032918    WO-A1-2015/047723**
**US-A- 4 249 907    US-A1- 2020 179 892**

- **CUELLAR E S ET AL: "Neuro-fuzzy architecture in a batch reactor temperature control system", INTELLIGENT CONTROL, 1994., PROCEEDINGS OF THE 1994 IEEE INTERNATIONAL SYMPOSIUM ON COLUMBUS, OH, USA 16-18 AUG. 1994, NEW YORK, NY, USA,IEEE, 16 August 1994 (1994-08-16), pages 11 - 15, XP010126500, ISBN: 978-0-7803-1990-5, DOI: 10.1109/ISIC.1994.367849**

**Beschreibung**

Die Erfindung betrifft ein Verfahren zur Regelung der Temperatur in einem verfahrenstechnischen Apparat, bei dem in einem Primärkreislauf eine Flüssigkeit aus dem Apparat gefördert, zumindest teilweise einem Wärmeübertrager zugeführt und zumindest teilweise dem Apparat wieder zurückgeführt wird.

Bekannte Regelungen der Temperatur in einem verfahrenstechnischen Apparat sind u.a. in der folgenden Veröffentlichung auf den Seiten 165 - 171 beschrieben: H. Schuler, Prozessführung, Oldenbourg Verlag München Wien, 1999. Hierbei werden zum Beispiel Temperaturen durch Sensoren oder Durchflussraten durch Ventilstellungen oder Durchflusssensoren ermittelt und für die Regelung als Reglereingangssignale verwendet.

Zur Regelung der Temperatur in einem Reaktor sind unterschiedliche Konzepte auf Basis von Wärmeübertragerelementen im Reaktor bekannt. Solche Wärmeübertragerelemente können u.a. Rohrwendeln sein oder einen Kühl- oder Heizmantel an der Außenseite des Reaktors umfassen. Bei manchen exothermen Reaktionen reicht jedoch ein Kühlmantel nicht aus, um die in der Reaktion freiwerdende Wärme abzuführen. In diesen Fällen wird das Reaktionsgemisch üblicherweise aus dem Reaktor in einen Primärkreislauf gefördert, der einen Wärmeübertrager und eine Rückführung in den Reaktor enthält. Der Wärmeübertrager führt die Reaktionswärme aus dem Reaktionsgemisch ab. Details zur Verwendung, Auslegung und Funktioneise von Wärmeübertragern werden u.a. in dem folgenden Dokument im Kapitel 8.29 offenbart: B. G. Liptak, Process Control and Optimization, Instrument Engineers' Handbook, Volume Two, Taylor & Francis Inc., 4. Edition, 2005.

In einer speziellen Anwendung für Hydroformylierungen offenbart das Dokument WO 2015/047723 A1 (THE DOW CHEMICAL COMPANY) eine Temperaturregelung für einen Reaktor, bei der die Temperatur im Reaktor durch einen Temperatursensor erfasst wird und ein Regelungsalgorithmus die Durchflussmenge durch einen Wärmeübertrager in einem Primärkreislauf so einstellt, dass die Reaktortemperatur auf ihrem Sollwert gehalten wird. In einer alternativen Variante umfasst der Primärkreislauf einen Bypass parallel zum Wärmeübertrager, sodass der Strom im Primärkreislauf in zwei parallele Teilströme aufgeteilt werden kann. Die Regelung der Reaktortemperatur kann in diesem Fall auch durch die Beeinflussung des Durchflusses durch den Bypass erfolgen.

Nachteilig an diesen offenbarten Temperaturregelungen ist die nicht-lineare Regelungscharakteristik, die schwache Regelgüte und auftretendes Schwingungsverhalten bei dynamischen Prozessen wie beispielsweise einer zeitlichen Laständerung oder einer Störgrößeneinwirkung.

Es stellte sich daher die Aufgabe, eine stabilere und präzisere Regelung für die Temperatur in einem verfahrenstechnischen Apparat bereitzustellen. Hierbei sollte auch die Regelgüte bei dynamischen Laständerungen und Störungen im Prozess verbessert werden.

Diese Aufgabe wurde erfindungsgemäß durch ein Verfahren gemäß Anspruch 1 gelöst. Vorteilhafte Ausgestaltungen des erfindungsgemäßen Verfahrens sind in den Ansprüchen 2 bis 12 angegeben.

Das erfindungsgemäße Verfahren zur Regelung der Temperatur in einem verfahrenstechnischen Apparat, bei dem in einem Primärkreislauf eine Flüssigkeit aus dem Apparat gefördert, zumindest teilweise einem Wärmeübertrager zugeführt und zumindest teilweise dem Apparat wieder zurückgeführt wird, wobei der Wärmeübertrager durch ein Wärmeträgermedium in einem Sekundärkreislauf gekühlt oder beheizt wird, umfasst die Schritte:

- Bereitstellen eines Sollwertes der Temperatur der Flüssigkeit im Apparat,
- Erfassen eines Istwertes der Temperatur der Flüssigkeit im Apparat,
- Berechnen der Temperaturdifferenz zwischen dem Istwert und dem Sollwert der Flüssigkeit im Apparat.

Erfindungsgemäß werden

- ein der Flüssigkeit im Primärkreislauf durch den Wärmeübertrager entnommener oder hinzugefügter Wärmestrom ermittelt,
- auf Basis eines vorgegebenen Regelungsalgorithmus ein Stellsignal berechnet, wobei der Regelungsalgorithmus derart konfiguriert ist, dass das Stellsignal sich in Abhängigkeit von dem Wärmestrom und der Temperaturdifferenz zwischen dem Istwert und dem Sollwert der Flüssigkeit im Apparat ergibt, und
- die Durchflussmenge des Stromes der Flüssigkeit durch den Wärmeübertrager im Primärkreislauf und/oder ein Mengenstrom des Wärmeträgermediums durch den Wärmeübertrager im Sekundärkreislauf auf Basis des Stellsignals manipuliert.

Es hat sich gezeigt, dass die Berücksichtigung des Wärmestroms bei der Regelung der Temperatur in einem verfahrenstechnischen Apparat sowohl eine stabilere Regelung als auch eine bessere Regelgüte ermöglicht.

Der Regelungsalgorithmus ist derart konfiguriert, dass sich das Stellsignal in Abhängigkeit von dem Wärmestrom und der Temperaturdifferenz zwischen dem Istwert und dem Sollwert der Flüssigkeit im Apparat ergibt. Im Sinne dieser Schrift ist dies gleichbedeutend damit, dass bei der Berechnung des Stellsignals der Wärmestrom und die Temperaturdifferenz zwischen dem Istwert und dem Sollwert der Flüssigkeit im Apparat berücksichtigt werden. In Bezug auf die Ermittlung des Stellsignals auf Basis des vorgegebenen Regelungsalgorithmus werden in dieser Schrift die Begriffe "berechnet" und "generiert" synonym verwendet.

Die Begriffe "berücksichtigt" und "Berücksichtigung" sind in Bezug auf den Regelungsalgorithmus in dieser Schrift so zu verstehen, dass eine durch den Regelungsalgorithmus berechnete Ausgangsgröße, beispielsweise ein Stellsignal, von der jeweils berücksichtigten Größe abhängig ist. Dabei sind die Begriffe nicht exklusiv zu verstehen. Die Berücksichtigung einer Größe X schließt nicht aus, dass auch eine Größe Y in dem Regelungsalgorithmus berücksichtigt wird. Beispielsweise ist die Berücksichtigung des Wärmestroms im Regelungsalgorithmus so zu verstehen, dass das Stellsignal in Abhängigkeit von dem Wert des Wärmestroms berechnet wird, und die Berücksichtigung der Temperaturdifferenz zwischen dem Istwert und dem Sollwert der Flüssigkeit im Apparat im Regelalgorithmus ist so zu verstehen, dass das Stellsignal in Abhängigkeit des Wertes der Temperaturdifferenz berechnet wird. Die Abhängigkeit kann beispielhaft durch eine mathematische Funktion f der Form "Stellsignal = f(Wärmestrom, Temperaturdifferenz zwischen dem Istwert und dem Sollwert der Flüssigkeit im Apparat)" ausgedrückt werden. Eine solche Funktion kann auch weitere Parameter oder Einflussgrößen neben dem Wärmestrom und der Temperaturdifferenz enthalten, beispielsweise Störgrößen.

Unter einem "verfahrenstechnischen Apparat" wird in dieser Schrift ein Apparat verstanden, der ein Flüssigkeitsvolumen aufnehmen kann und zum Durchströmen mit einer Flüssigkeit geeignet ist, beispielsweise ein Reaktor, ein Rührbehälter, eine Kolonne, ein Phasenscheider oder ein Tank. In bevorzugten Ausführungsformen des erfindungsgemäßen Verfahrens ist der verfahrenstechnische Apparat ein Reaktor.

Unter einem "Wärmeübertrager" wird in dieser Schrift ein Apparat verstanden, der Wärme von einem Medium auf ein anderes Medium übertragen kann. Beispielhaft können Plattenwärme-übertrager oder Rohrbündelwärmeübertrager in dem erfindungsgemäßen Verfahren eingesetzt werden.

Ein "Primärkreislauf" stellt in dieser Schrift einen Kreislaufstrom dar, bei dem eine Flüssigkeit aus dem verfahrenstechnischen Apparat gefördert, zumindest teilweise einem Wärmeübertrager zugeführt und zumindest teilweise dem Apparat wieder zurückgeführt wird. Der Primärkreislauf kann auch Verzweigungen enthalten, beispielsweise als Bypass parallel zum verfahrenstechnischen Apparat. Es können auch mehrere Wärmetauscher in dem Primärkreislauf vorhanden sein, beispielsweise als Parallelschaltungen, Serienschaltungen oder Kombinationen von Parallel- und Serienschaltungen.

Ein "Regelungsalgorithmus" im Sinne dieser Schrift generiert ein Stellsignal zur Manipulation der Durchflussmenge des Flüssigkeitsstroms durch den Wärmeübertrager im Primärkreislauf in Abhängigkeit von einer oder mehreren Regelabweichungen, unter anderem der Temperaturdifferenz zwischen dem Istwert und dem Sollwert der Temperatur der Flüssigkeit im Apparat. Erfindungsgemäß generiert der Regelungsalgorithmus das Stellsignal zusätzlich in Abhängigkeit von dem durch einen Wärmeübertrager entnommenen oder hinzugefügten Wärmestrom. Der Wärmestrom stellt, ebenso wie die Temperaturdifferenz zwischen dem Istwert und dem Sollwert der Flüssigkeit im Apparat, eine Einflussgröße für den Regelungsalgorithmus dar. Weitere Einflussgrößen können in dem Regelungsalgorithmus zur Berechnung des Stellsignals berücksichtigt werden, beispielsweise Eduktmengenströme oder Größen, die eine chemische Umsetzung im Apparat repräsentieren.

Der Regelungsalgorihmus kann sich durch eine direkte elektronische Verschaltung von Hardwarekomponenten ergeben. Beispiele für Hardwarekomponenten sind PID-Regler, Temperatursensoren, Durchflusssensoren und Stellorgane zur Einstellung des Mengenstroms des Wärmeträgermediums im Sekundärkreislauf oder des Durchflusses des Stromes der Flüssigkeit im Primärkreislauf. Der Regelungsalgorihmus kann sich ferner auch durch ein Zusammenspiel zwischen Hardware- und Softwarekomponenten ergeben, wobei die Softwarekomponenten auf Basis von Eingangsgrößen ein oder mehrere Ausgangssignale durch computerimplementierte, numerische Algorithmen berechnen. Das Ausgangssignal kann beispielhaft das Stellsignal für ein Stellorgan sein, welches z.B. den Durchfluss des Stromes der Flüssigkeit einstellt.

Die konkrete Ausgestaltung, wie die einzelnen Einflussgrößen miteinander zusammenhängen und wie die Einflussgrößen das Stellsignal im Detail generieren, kann durch beispielsweise einen mathematischen Zusammenhang, bevorzugt durch eine mathematische, algebraische Funktion, oder durch partielle oder gewöhnliche Differentialgleichungen, beschrieben werden.

In vielen Fällen werden verfahrenstechnische Prozesse durch ein Prozessleitsystem geregelt und/oder gesteuert, welche bevorzugt Eingrößen-Regler einsetzen. Die Eingrößen-Regler erhalten ein Eingangssignal und geben entsprechend ihres Regelungsalgorithmus ein Ausgangssignal aus. So kann bevorzugt ein Eingrößen-Regler auch ein PID-Regler sein.

Im Folgenden sind die mathematischen Zusammenhänge für den PID-Regler beispielhaft dargelegt.

$$y(t) = K_P \left( e(t) + \frac{1}{T_i} \int_0^t e(\tau)\, d\tau \right) + T_d \frac{de(t)}{dt}$$

Hierbei sind y(t) der Reglerausgang in Abhängigkeit von der Zeit $t$, $e(t)$ die Regelabweichung in Abhängigkeit von der Zeit $t$, $K_P$ die Reglerverstärkung, welche den proportionalen Anteil der Reglerfunktion repräsentiert, $T_i$ die Integrationszeitkonstante, welche den integralen Anteil der Reglerfunktion repräsentiert, $\tau$ die Integrationsvariable und $T_d$ die Ableitungszeitkonstante, welche den differentiellen Anteil der Reglerfunktion repräsentiert. Die Regelabweichung $e(t)$ berechnet sich als Differenz zwischen dem Sollwert w(t) und dem Istwert x(t) zu $e(t) = w(t) - x(t)$.

Der Regelungsalgorithmus kann auch ein adaptiver Regelungsalgorithmus sein. Beispielsweise kann der Regelungsalgorithmus so konfiguriert sein, dass ein Parameter des Regelungsalgorithmus, z.B. die Reglerverstärkung $K_P$, nicht fest vorgegeben ist, sondern sein Wert über die Zeit in Abhängigkeit von bestimmten Prozessgrößen berechnet wird. Beispielsweise kann die Reglerverstärkung $K_P$ als Funktion des Wärmekapazitätsstroms auf der Primärseite und/oder der Sekundärseite des Wärmetauschers formuliert werden, um Nichtlinearitäten der Wärme-übertragercharakteristik zu kompensieren. Die Nichtlinearitäten können für die unterschiedlichen Strömungsführungen aus bekannter Fachliteratur entnommen werden, beispielsweise aus dem VDI-Wärmeatlas (VDI-Wärmeatlas, Springer Vieweg, Berlin, Heidelberg, 12. Auflage, 2019, ISBN 978-3-662-52988-1, eBook: 978-3-662-52989-8).

Unter einer "Störgröße" wird in dieser Schrift eine gemessene oder geschätzte Prozessgröße verstanden, die störend auf die zu regelnde Größe einwirkt und nicht durch den Regelungsalgorithmus beeinflusst wird. Beispielsweise kann dies ein Rohstoffstrom in den verfahrenstechnischen Apparat oder ein hinzugefügter oder entnommener Wärmestrom sein.

Eine "Störgrößenaufschaltung" bedeutet in dieser Schrift, dass der Regelungsalgorithmus eine Störgröße bei der Berechnung eines Stellsignals berücksichtigt.

Unter einer "Flüssigkeit" wird in dieser Schrift ein einphasiges oder mehrphasiges Fluid verstanden. Die Flüssigkeit ist somit fließfähig und durch den Primärkreislauf förderbar. Die Flüssigkeit kann auch Gasanteile und/oder Feststoffanteile enthalten, insofern solch eine Flüssigkeit noch förderbar ist.

Unter dem Begriff "Flüssigkeitsstrom" wird in dieser Schrift der "Durchfluss der Flüssigkeit" im Sinne der "Durchflussmenge des Stromes der Flüssigkeit" verstanden, sofern sich aus dem Zusammenhang nicht eine abweichende Bedeutung ergibt. Mithin werden diese drei Begrifflichkeiten in dieser Schrift austauschbar verwendet.

Unter einem "Wärmeträgermedium" wird in dieser Schrift ein einphasiges oder mehrphasiges Fluid verstanden, beispielsweise eine Flüssigkeit, ein Gas, ein Dampf oder Gemische hieraus. Das Wärmeträgermedium ist somit fließfähig und durch den Sekundärkreislauf förderbar. Das Wärmeträgermedium kann auch Feststoffanteile enthalten, insofern solch ein Wärmeträgermedium noch förderbar ist. In einigen Anwendungsfällen ist das Wärmeträgermedium vorzugsweise Heizdampf, Luft oder gefiltertes Flusswasser.

In einer bevorzugten Ausgestaltung des erfindungsgemäßen Verfahrens wird im Regelungsalgorithmus eine Wärmestromdifferenz zwischen dem ermittelten Wärmestrom als Istwert und einem fest oder im zeitlichen Verlauf änderbaren vorgegebenen Sollwert des Wärmestroms ermittelt. Auf Basis dieser Wärmestromdifferenz und der ermittelten Temperaturdifferenz zwischen dem Istwert und dem Sollwert der Temperatur der Flüssigkeit im Apparat wird ein Stellsignal berechnet, auf Basis dessen die Durchflussmenge des Stromes der Flüssigkeit durch den Wärmeübertrager im Primärkreislauf und/oder ein Mengenstrom des Wärmeträgermediums durch den Wärmeübertrager im Sekundärkreislauf manipuliert wird.

In einer bevorzugten Ausgestaltung des erfindungsgemäßen Verfahrens wird der Wärmestrom aus dem Flüssigkeitsstrom durch den Wärmeübertrager im Primärkreislauf sowie aus einer Temperaturdifferenz zwischen der Temperatur der Flüssigkeit vor und nach dem Wärmeübertrager im Primärkreislauf ermittelt. In einer alternativen bevorzugten Ausgestaltung wird der Wärmestrom aus einem Mengenstrom des Wärmeträgermediums durch den Wärmeübertrager im Sekundärkreislauf sowie aus einer Temperaturdifferenz zwischen der Temperatur des Wärmeträgermediums vor und nach dem Wärmeübertrager im Sekundärkreislauf ermittelt. Diese beiden bevorzugten Ausgestaltungen können auch kombiniert werden, sodass der Wärmestrom aus einem Flüssigkeitsstrom durch den Wärmeübertrager im Primärkreislauf sowie aus einer Temperaturdifferenz zwischen der Temperatur der Flüssigkeit vor und nach dem Wärmeübertrager im Primärkreislauf und aus einem Mengenstrom des Wärmeträgermediums durch den Wärmeübertrager im Sekundärkreislauf sowie aus einer Temperaturdifferenz zwischen der Temperatur des Wärmeträgermediums vor und nach dem Wärmeübertrager im Sekundärkreislauf ermittelt wird.

Die Ermittlung des Wärmestroms gemäß dieser Ausgestaltung hat den Vorteil der leichten messtechnischen Umsetzbarkeit. Bewährte und zuverlässige Sensoren für Durchfluss und Temperatur sind für unterschiedliche Anwendungsfälle kommerziell verfügbar, sodass gemäß dieser Ausgestaltung des erfindungsgemäßen Verfahrens eine einfache und zuverlässige Ermittlung des Wärmestroms realisiert werden kann. Zudem können durch die Erfassung der zur Ermittlung verwendeten Durchflüsse und Temperaturen in der Nähe des Wärmeübertragers gegebenenfalls dort auftretende Störungen oder Schwankungen schnell erfasst und bei der Temperaturregelung berücksichtigt werden, bevor sich die Störungen oder Schwankungen auf die Temperatur im verfahrenstechnischen Apparat auswirken.

In einer weiteren bevorzugten Ausgestaltung des erfindungsgemäßen Verfahrens umfasst der Regelungsalgorithmus einen Temperaturregler und einen Wärmestromregler, wobei der Temperaturregler in Abhängigkeit von der Temperaturdifferenz zwischen dem Istwert und dem Sollwert der Flüssigkeit im Apparat als Ausgangssignal einen Sollwert für den der Flüssigkeit im Primärkreislauf durch den Wärmeübertrager entnommenen oder hinzugefügten Wärmestrom berechnet, dieser Sollwert an den Wärmestromregler übergeben wird, und der Wärmestromregler in Abhängigkeit von der Differenz zwischen dem Istwert und dem Sollwert des Wärmestroms als Ausgangssignal das Stellsignal zur Manipulation des Flüssigkeitsstroms im Primärkreislauf und/oder zur Manipulation des Mengenstroms des Wärmeträgermediums durch den Wärmeübertrager im Sekundärkreislauf generiert. Diese Ausgestaltung des Regelungsalgorithmus entspricht einer Kaskadenregelung mit dem Temperaturregler als Führungsregler und dem Wärmestromregler als Folgeregler.

In einer vorteilhaften Ausgestaltung der Kaskadenregelung werden der Führungsregler und der Folgeregler jeweils als PID-Regler ausgeführt. Zum Aufbau der Kaskade wird der Reglerausgang $y_1(t)$ des Führungsreglers gleich dem Sollwert $w_2(t)$ des Folgereglers gesetzt. Die Istwerte ergeben sich aus der Messung der Temperatur im Apparat und dem ermittelten Wärmestrom.

In einer besonders bevorzugten Ausgestaltung der Kaskadenregelung sind in dem Führungsregler und dem Folgeregler folgende Gleichungen implementiert:

$$y_1(t) = w_2(t) = \dot{Q}_{HE,soll}$$

$$= K_{P,1}\left(T_{R,soll}(t) - T_R(t) + \frac{1}{T_{i,1}}\int_0^t \left(T_{R,soll}(\tau) - T_R(\tau)\right)d\tau\right)$$

$$+ T_{d,1}\frac{d\left(T_{R,soll}(t) - T_R(t)\right)}{dt}$$

$$y_2(t) = \dot{m}_{HE,soll}$$

$$= K_{P,2}\left(\dot{Q}_{HE,soll}(t) - \dot{Q}_{HE}(t) + \frac{1}{T_{i,2}}\int_0^t \left(\dot{Q}_{HE,soll}(\tau) - \dot{Q}_{HE}(\tau)\right)d\tau\right)$$

$$+ T_{d,2}\frac{d\left(\dot{Q}_{HE,soll}(t) - \dot{Q}_{HE}(t)\right)}{dt}$$

Hierbei sind $\dot{Q}_{HE,soll}$ der Sollwert des Wärmestroms, der durch den Führungsregler bestimmt wird, $K_{P,1}$ die Reglerverstärkung des Führungsreglers für die Temperatur der Flüssigkeit im Apparat, $T_{R,soll}$ der Sollwert der Temperatur der Flüssigkeit im Apparat, $T_R$ der Istwert der Temperatur der Flüssigkeit im Apparat, $T_{i,1}$ die Integrationszeitkonstante des Führungsreglers für die Temperatur der Flüssigkeit im Apparat, $T_{d,1}$ die Ableitungszeitkonstante des Führungsreglers für die Temperatur der Flüssigkeit im Apparat, $\dot{m}_{HE,soll}$ der Sollwert des geregelten Massenstroms durch den Wärmeübertrager, $K_{P,2}$ die Reglerverstärkung des Folgereglers für den Wärmestrom, $\dot{Q}_{HE}$ der Istwert des Wärmestroms, $T_{i,2}$ die Integrationszeitkonstante des Folgereglers für den Wärmestrom und $T_{d,2}$ die Ableitungszeitkonstante des Folgereglers für den Wärmestrom.

Es hat sich herausgestellt, dass der der Flüssigkeit im Primärkreislauf durch den Wärmeübertrager entnommene oder hinzugefügte Wärmestrom schneller auf Stelleingriffe des Regelungsalgorithmus oder Störungen reagiert als die in dem verfahrenstechnischen Apparat messbare Temperatur. Eine Kaskadierung von Temperaturregler als Führungsregler und Wärmestromregler als Folgeregler hat den Vorteil, dass die Unterschiede in den Dynamiken der Teilregelkreise genutzt werden, um die Soll-Temperatur im verfahrenstechnischen Apparat bei Sollwertänderungen oder Störungen schneller und präziser einzustellen. Die Regelgüte des Regelungsalgorithmus wird somit verbessert.

In einer bevorzugten Ausgestaltung ist im Primärkreislauf zumindest ein Bypass parallel zu dem Wärmeübertrager geschaltet. Hierdurch kann der Flüssigkeitsstrom im Primärkreislauf flexibler gestaltet werden. Dies ist beispielsweise dann von Vorteil, wenn es technische oder prozessbedingte Restriktionen gibt, die sich auf den Flüssigkeitsstrom im Primärkreislauf auswirken. So kann zum Beispiel beim Einsatz eines kosteneffizienten Luftkühlers als Wärmeübertrager üblicherweise die Kühlleistung nur stufenweise oder nur auf einen konstanten Wert eingestellt werden. In einem solchen Fall, bei dem der Luftkühler konstant betrieben wird, hat ein Bypass im Primärkreislauf auch den Vorteil, dass der Durchfluss der Flüssigkeit durch den Wärmeübertrager sehr einfach geregelt werden kann, indem die Regelung der Temperatur im verfahrenstechnischen Apparat durch die Manipulation des Flüssigkeitsstromes durch den Bypass erfolgen kann.

Die Manipulation des Flüssigkeitsstroms durch den Wärmeübertrager im Primärkreislauf kann auf unterschiedliche Arten realisiert werden, beispielsweise direkt, indirekt oder durch eine Kombination von direkter und indirekter Manipulation.

Bei einer direkten Manipulation des Flüssigkeitsstroms durch den Wärmeübertrager ist bevorzugt im Primärkreislauf in der Zuleitung zum Wärmeübertrager oder in der Ableitung vom Wärmeübertrager oder sowohl in der Zuleitung zum Wärmeübertrager als auch in der Ableitung vom Wärmeübertrager mindestens ein Stellorgan und/oder eine Pumpe zur Manipulation des Flüssigkeitsstromes auf Basis des Stellsignals vorhanden.

Bei einer indirekten Manipulation des Flüssigkeitsstroms durch den Wärmeübertrager ist bevorzugt in einem Bypass, der parallel zum Wärmeübertrager geschaltet ist, mindestens ein Stellorgan und/oder eine Pumpe zur Manipulation des Flüssigkeitsstroms durch den Wärmeübertrager auf Basis des Stellsignals vorhanden.

Bei einer Kombination von direkter und indirekter Manipulation ist bevorzugt in einem Bypass, der parallel zum Wärmeübertrager geschaltet ist, mindestens ein Stellorgan zur Manipulation des Flüssigkeitsstromes auf Basis des Stellsignals vorhanden. Ferner ist bei dieser Variante im Primärkreislauf in der Zuleitung zum Wärmeübertrager oder in der Ableitung vom Wärmeübertrager oder sowohl in der Zuleitung zum Wärmeübertrager als auch in der Ableitung vom Wärmeübertrager mindestens ein Stellorgan zur Manipulation des Flüssigkeitsstroms auf Basis des Stellsignals vorhanden.

Zur Manipulation des Mengenstroms des Wärmeträgermediums durch den Wärmeübertrager im Sekundärkreislauf auf Basis des Stellsignals ist bevorzugt in der Zuleitung zum Wärmeübertrager oder in der Ableitung vom Wärmeübertrager oder sowohl in der Zuleitung zum Wärmeübertrager als auch in der Ableitung vom Wärmeübertrager mindestens ein Stellorgan vorhanden.

Geeignete Stellorgane sind dem Fachmann bekannt. Vorzugsweise ist das Stellorgan ein Regelventil, das geeignet ist, eine Durchflussmenge schnell und präzise einzustellen.

Erfindungsgemäß wird der Flüssigkeit im Primärkreislauf durch einen Wärmeübertrager ein Wärmestrom entnommen oder hinzugefügt. Ferner wird der Flüssigkeitsstrom durch den Wärmeübertrager und/oder der Mengenstrom des Wärmeträgermediums im Sekundärkreislauf durch ein Stellsignal manipuliert. Die Erfindung ist jedoch nicht auf genau einen Wärmeübertrager beschränkt. Von der Erfindung erfasst sind auch Ausführungsformen, bei denen mehrere Wärmeübertrager im Primärkreislauf vorhanden sind. Diese können in Parallelschaltungen, Serienschaltungen oder Kombinationen von Parallel- und Serienschaltungen angeordnet sein. Dabei wird der Flüssigkeitsstrom durch mindestens einen Wärmeübertrager manipuliert. Es können jedoch auch Flüssigkeitsströme durch mehrere Wärmeübertrager durch das erfindungsgemäße Verfahren manipuliert werden.

In einer Ausgestaltung ist ein weiterer Wärmeübertrager im Primärkreislauf parallel zum ersten Wärmeübertrager geschaltet, sodass der verfahrenstechnische Apparat bei Bedarf von zwei Wärmeübertragern temperiert werden kann. Eine derartige Ausgestaltung erhöht die Flexibilität bezüglich der Bereitstellung von Wärmeleistung oder Kühlleistung. Ein Anwendungsbeispiel, bei dem diese Flexibilität wirtschaftlich vorteilhaft ausgenutzt werden kann, ist die Einkopplung von Wärmeleistung oder Kühlleistung aus anderen Prozessstufen oder anderen Anlagen, die ansonsten ungenutzt bleiben würde und im Rahmen der erfindungsgemäßen Regelung nicht beeinflussbar ist.

In einer weiteren Ausgestaltung ist im Primärkreislauf in der Zuleitung zum ersten und zum zweiten Wärmeübertrager mindestens ein Stellorgan und/oder eine Pumpe zur Manipulation des Flüssigkeitsstromes auf Basis des Stellsignals vorhanden.

In einer weiteren Ausgestaltung ist im Primärkreislauf in der Zuleitung zum ersten und zum zweiten Wärmeübertrager und in einem zu den Wärmeübertragern parallelgeschalteten Bypass mindestens ein Stellorgan und/oder eine Pumpe zur Manipulation des Flüssigkeitsstromes auf Basis des Stellsignals vorhanden.

Bei den Ausgestaltungen, welche in einem Bypass ein Stellorgan besitzen und/oder welche mindestens zwei Wärmeübertrager mit ihnen jeweils zugeordneten Stellorganen besitzen, kann der Flüssigkeitsstrom durch den jeweiligen Wärmeübertrager begrenzt werden. Dies ist besonders dann ein Vorteil, wenn beispielsweise eine Pumpe für den Primärkreislauf nur einen konstanten Flüssigkeitsstrom fördern kann.

Bei einer Ausgestaltung, bei der der Flüssigkeitsstrom im Primärkreislauf durch eine Pumpe mit variabler Fördermenge gefördert wird, kann diese Pumpe vorteilhaft zur Manipulation der Durchflussmenge des Stromes der Flüssigkeit durch den Wärmeübertrager auf Basis des Stellsignals verwendet werden, beispielsweise indem das Stellsignal die Drehzahl der Pumpe vorgibt.

In einer weiteren Ausgestaltung umfasst der Regelungsalgorithmus eine Split-Range-Regelung, bei der anstatt eines Stellsignals mindestens zwei Stellsignale berechnet und mindestens zwei Ströme im Primärkreislauf und/oder im Sekundärkreislauf manipuliert werden, wobei ein erster Strom in Abhängigkeit von einem ersten Stellsignal und ein zweiter Strom in Abhängigkeit von einem zweiten Stellsignal manipuliert werden.

In einer weiteren Ausgestaltung umfasst der Regelungsalgorithmus eine Split-Range-Regelung, bei der mindestens zwei Stellsignale berechnet und mindestens zwei Ströme im Primärkreislauf manipuliert werden, wobei ein erster Flüssigkeitsstrom in Abhängigkeit von einem ersten Stellsignal und ein zweiter Flüssigkeitsstrom in Abhängigkeit von einem zweiten Stellsignal manipuliert werden.

In einer weiteren Ausgestaltung umfasst der Regelungsalgorithmus eine Split-Range-Regelung, bei der mindestens zwei Stellsignale berechnet und mindestens zwei Ströme im Sekundärkreislauf manipuliert werden, wobei ein erster Mengenstrom in Abhängigkeit von einem ersten Stellsignal und ein zweiter Mengenstrom in Abhängigkeit von einem zweiten Stellsignal manipuliert werden.

In einer weiteren Ausgestaltung umfasst der Regelungsalgorithmus eine Split-Range-Regelung, bei der mindestens zwei Stellsignale berechnet und mindestens zwei Ströme, davon einer im Primärkreislauf und einer im Sekundärkreislauf, manipuliert werden, wobei ein Flüssigkeitsstrom im Primärkreislauf in Abhängigkeit von einem ersten Stellsignal und ein Mengenstrom im Sekundärkreislauf in Abhängigkeit von einem zweiten Stellsignal manipuliert werden.

Ausgestaltungen mit Split-Range-Regelungen sind insbesondere dann von Vorteil, wenn Stellorgane mit unterschiedlichen Nennweiten und somit signifikant unterschiedlichen Durchflussmengen vorhanden sind. In einem solchen Fall lässt sich eine präzise Steuerung des Mengenstroms des Wärmeträgermediums gewährleisten, indem ein großes Stellorgan, welches für große Mengenströme ausgelegt ist, und ein kleines Stellorgan, welches für kleine Mengenströme ausgelegt ist, in einer Parallelschaltung angeordnet sind.

In einer bevorzugten Ausgestaltung des erfindungsgemäßen Verfahrens, bei der im Primärkreislauf zumindest ein Bypass parallel zu einem Wärmeübertrager geschaltet ist, wird die aus dem verfahrenstechnischen Apparat in den Primärkreislauf geförderte Flüssigkeitsmenge auf einen vorgegebenen Sollwert eingestellt, indem der Istwert der Flüssigkeitsmenge ermittelt wird, aus dem Vergleich zwischen Istwert und Sollwert ein Ausgangssignal berechnet wird, und der durch den Bypass strömende Flüssigkeitsstrom durch das Ausgangssignal manipuliert wird. Diese Ausgestaltung ist insbesondere dann vorteilhaft, wenn der aus dem verfahrenstechnischen Apparat in den Primärkreislauf geförderte Flüssigkeitsstrom konstant gehalten werden soll oder muss, zugleich aber eine effiziente Regelung der Temperatur in dem Apparat angestrebt wird.

In einer weiteren Ausgestaltung berücksichtigt der Regelungsalgorithmus bei der Berechnung des Stellsignals mindestens eine Störgröße, wobei die Störgröße eine gemessene oder geschätzte Prozessgröße umfasst. So kann die Störgröße beispielsweise einen Rohstoffstrom darstellen, der in den verfahrenstechnischen Apparat geleitet wird. Die Störgröße kann auch beispielsweise einen oder mehrere weitere Wärmeströme umfassen. Hierbei ergibt sich u.a. der Vorteil, dass die Regelung bei beispielsweise einer bevorstehenden Änderung der freigesetzten oder verbrauchten Wärme im Apparat aufgrund von geänderten Eingangsströmen rechtzeitig reagieren kann. Es können auch mehrere oder kombinierte Störgrößen beachtet werden.

In einer weiteren Ausgestaltung läuft in dem verfahrenstechnischen Apparat eine chemische oder biologische, endotherme oder exotherme Reaktion oder ein exothermer oder endothermer physikalischer Prozess ab.

In einer bevorzugten Ausgestaltung handelt es sich bei der Reaktion um eine Hydroformylierung, Veretherung, Etherrückspaltung, Enalisierung, Dehydrierung, Pyrolyse, Hydrierung oder einen Crackprozess.

In einer besonders bevorzugten Ausgestaltung ist die Hydrierung eine Vollhydrierung, Selektivhydrierung oder Kernhydrierung.

Bei derartigen Reaktionen und Prozessen können große Laständerungen oder Beeinflussungen durch Störgrößen auftreten. Das erfindungsgemäße Verfahren gewährleistet insbesondere bei solch anspruchsvollen Regelungsaufgaben eine schnelle und robuste Einstellung der gewünschten Temperatur in dem verfahrenstechnischen Apparat.

In einer weiteren Ausgestaltung handelt es sich bei dem Regelungsalgorithmus um einen Mehrgrößenregler, bevorzugt einen Modellprädiktiven Regler oder einen Zustandsraumregler. Hierdurch ergibt sich u.a. der Vorteil, dass mehrere physikalischen Größen im Verfahren gleichzeitig erfasst und zur Regelung verwendet werden können.

Die Erfindung wird im Folgenden mit Verweis auf die Zeichnungen näher erläutert. Die Zeichnungen sind als Prinzipdarstellungen zu verstehen. Sie stellen keine Beschränkung der Erfindung dar, beispielsweise im Hinblick auf konkrete Abmessungen oder Ausgestaltungsvarianten. Es zeigen:

Fig. 1: Verfahrenstechnischer Apparat mit externen Wärmeübertragern und Regelstruktur zur Regelung der Reaktortemperatur gemäß Stand der Technik

Fig. 2: Verfahrenstechnischer Apparat mit externen Wärmeübertragern und Regelstruktur zur Regelung der Reaktortemperatur gemäß einer ersten erfindungsgemäßen Ausführungsform

Fig. 3: Verfahrenstechnischer Apparat mit externen Wärmeübertragern und Regelstruktur zur Regelung der Reaktortemperatur gemäß einer zweiten erfindungsgemäßen Ausführungsform

Fig. 4: Verfahrenstechnischer Apparat mit externen Wärmeübertragern und Regelstruktur zur Regelung der Reaktortemperatur gemäß einer dritten erfindungsgemäßen Ausführungsform

Fig. 5: Verfahrenstechnischer Apparat mit externen Wärmeübertragern und Regelstruktur zur Regelung der Reaktortemperatur gemäß einer vierten erfindungsgemäßen Ausführungsform

Fig. 6: Führungsverhalten bei Sollwertänderung um 1°C für Beispiel 1 bei Regelung entsprechend dem Stand der Technik mit Temperatur-Temperatur-Kaskade

Fig. 7: Führungsverhalten bei Sollwertänderung um 1°C für Beispiel 1 bei erfindungsgemäßer Regelung mit Temperatur-Wärmestrom-Kaskade

Fig. 8: Störungsverhalten bei Änderung des Propenzulaufs um 10 % für Beispiel 1 bei Regelung entsprechend dem Stand der Technik mit Temperatur-Temperatur-Kaskade

Fig. 9: Störungsverhalten bei Änderung des Propenzulaufs um 10 % für Beispiel 1 bei erfindungsgemäßer Regelung mit Temperatur-Wärmestrom-Kaskade

Fig. 10: Störungsverhalten bei Änderung des abzuziehenden Wärmestroms durch eine Wärmeintegration um 10 % für Beispiel 1 bei Regelung entsprechend dem Stand der Technik mit Temperatur-Temperatur-Kaskade.

Fig. 11: Störungsverhalten bei Änderung des abzuziehenden Wärmestroms durch eine Wärmeintegration um 10 % für Beispiel 1 bei erfindungsgemäßer Regelung mit Temperatur-Wärmestrom-Kaskade.

Liste der verwendeten Bezugszeichen:

| 101 | Verfahrenstechnischer Apparat |
|---|---|
| 102 | Primärkreislauf |
| 103 | Wärmeübertrager |
| 104 | Sekundärkreislauf |
| 105 | Temperaturregler mit Temperatursensor im verfahrenstechnischen Apparat |
| 106 | Wärmestromfolgeregler |
| 108, 109 | Stellorgan |
| 110 | Pumpe |
| 111 | Leitung zur Materialrückgewinnung |
| 112 | Temperatursensor in der Rückführung des Primärkreislaufs zum verfahrenstechnischen Apparat |
| 113 | Temperaturfolgeregler beinhaltend einen Temperatursensor in der Rückleitung des Wärmeübertragers |
| 120 | Leitung (Synthesegas) |
| 121 | Leitung (Propen) |
| 122 | Leitung (Lösungsmittel) |
| 123 | Leitung für den Austrag des Zielprodukts |
| 124 | Regler für den Flüssigkeitspegel im verfahrenstechnischen Apparat |
| 125 | Druckregler |
| 126, | 127, 128, 129, 130, 140 Stellorgan |
| 141 | externe Vorgabe eines Drucksignals |
| 142 | Wärmeübertrager |
| 143 | Durchflussregler für die Einstellung des Flüssigkeitsstroms durch den Wärmeübertrager |
| 144 | Sekundärkreislauf |
| 145 | Temperatursensor nach dem Wärmeübertrager |

| 201 | Verfahrenstechnischer Apparat |
|---|---|
| 202 | Primärkreislauf |
| 203 | Wärmeübertrager |
| 204 | Sekundärkreislauf |
| 205 | Temperaturregler mit Temperatursensor im verfahrenstechnischen Apparat |
| 206 | Wärmestromfolgeregler |
| 208 | Stellorgan |
| 209 | Großes Stellorgan zur Einstellung des Wärmeträgermedium-Stroms des Wärmeübertragers |
| 210 | Pumpe |
| 211 | Leitung (Abgas) |
| 212 | Temperatursensor in der Rückleitung des Wärmeübertragers |
| 214 | Kleines Stellorgan |
| 220 | Leitung für Inertgas (Stickstoff) |
| 221 | Leitung (Aldehyd) |
| 222 | Leitung (Wasser mit Katalysator) |
| 223 | Leitung für den Austrag des Zielprodukts |
| 224 | Regler für den Flüssigkeitspegel (Wasser) im Phasenscheider |
| 226, 227, 228, 229, 230 | Stellorgan |
| 231 | Leitung für das Abwasser |
| 232 | Regler für den Flüssigkeitspegel (Zielprodukt) im Phasenscheider |
| 233 | Stellorgan |
| 234 | Split-Range Regler |
| 240 | Phasenscheider |
| 243 | Druckregler für die Regelung des Abgasstroms |

| 301 | Verfahrenstechnischer Apparat |
|---|---|
| 302 | Primärkreislauf |
| 303 | Wärmeübertrager |
| 304 | Sekundärkreislauf |
| 305 | Temperaturregler mit Temperatursensor im verfahrenstechnischen Apparat |
| 306 | Wärmestromfolgeregler |
| 307 | Bypass |
| 309 | Stellorgan |
| 310 | Pumpe |
| 311 | Leitung für das Abgas |
| 312 | Temperatursensor in der Rückleitung des Wärmeübertragers |
| 315 | Temperatursensor direkt vor der Rückführung des Flüssigkeitsstroms in den verfahrenstechnischen Apparat |
| 316 | Stellorgan |
| 320 | Leitung (Wasserstoff) |
| 321 | Leitung (Alken) |
| 323 | Leitung (Roh-Alkohol) |
| 324 | Regler für den Flüssigkeitspegel im verfahrenstechnischen Apparat |
| 325 | Druckregler |
| 326, 327, 329, 330 | Stellorgan |
| 343 | Durchflussregler, welcher den Massestrom (Roh-Alkohol) vorgibt |
| | |
| 401 | Verfahrenstechnischer Apparat |
| 402 | Primärkreislauf |
| 403 | Wärmeübertrager |
| 404 | Sekundärkreislauf |
| 405 | Temperaturregler mit Temperatursensor im verfahrenstechnischen Apparat |
| 406 | Wärmestromfolgeregler |
| 407 | Bypass |
| 408, 409 | Stellorgan |
| 410 | Pumpe |
| 412 | Temperatursensor in der Rückleitung des Wärmeübertragers |
| 415 | Temperatursensor unmittelbar vor der Rückführung des Flüssigkeitsstroms in den verfahrenstechnischen Apparat |
| 416 | Stellorgan |
| 417 | Störgrößenberechnung |
| 420 | Leitung (Ammoniumnitrat) |
| 421 | Leitung (Wasser) |
| 423 | Leitung für den Austrag des Zielprodukts |
| 424 | Regler für den Flüssigkeitspegel im verfahrenstechnischen Apparat |
| 426, 427, 430 | Stellorgan |
| 435 | Druckregler für die Regelung des Wärmeträgermedium-Stroms für den Wärmeübertrager |

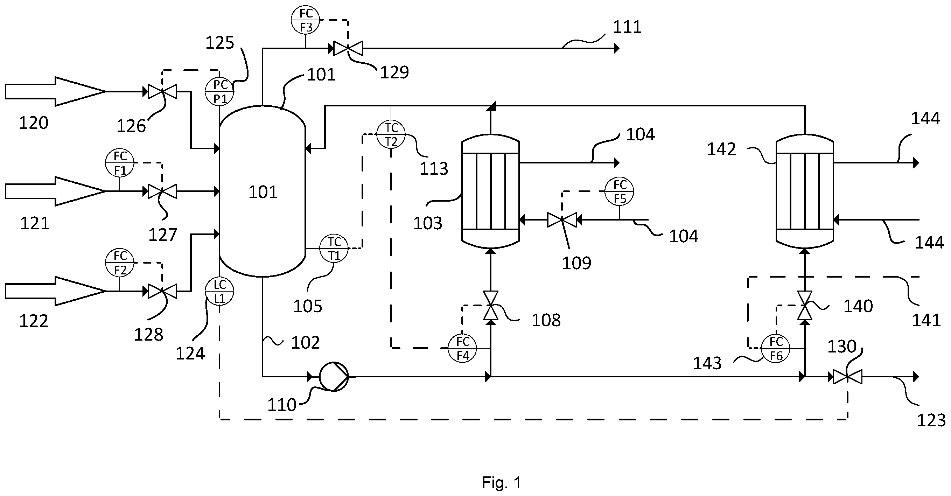

Fig. 1 zeigt ein Prozessfließbild eines verfahrenstechnischen Prozesses gemäß dem Stand der Technik, bei dem die Temperatur in einem Reaktor 101 als verfahrenstechnischer Apparat über externe Wärmeübertrager 103, 142 eingestellt wird. Dem Reaktor 101 werden über separate Leitungen Einsatzstoffe zugeführt, die im Reaktor zu einem oder mehreren Produkten umgesetzt werden. In dem dargestellten Beispiel werden über die Leitung 120 ein gasförmiger Einsatzstoff und über die Leitungen 121 und 122 flüssige oder gasförmige Einsatzstoffe zugeführt. Die Zufuhr der Einsatzstoffe in den Leitungen 121 und 122 erfolgt mengengeregelt über zwei Regelventile 127, 128 in den Zuführleitungen. Die Menge an zugeführtem gasförmigem Einsatzstoff in der Leitung 120 wird über einen Druckregler 125 und ein Regelventil 126 in der Zuführleitung so eingestellt, dass ein vorgegebener Druck im Reaktor 101 gehalten wird.

Gasförmige Reaktionsprodukte sowie gegebenenfalls nicht umgesetzte gasförmige Einsatzstoffe werden über einen gasförmigen Reaktoraustrag 111 aus dem Reaktor entfernt. Im dargestellten Beispiel erfolgt der Austrag mengengeregelt über ein Regelventil 129 im Reaktoraustrag 111. Flüssige Reaktionsprodukte werden über eine Pumpe 110 zwangsgefördert in einen Primärkreislauf 102 ausgetragen, der den Reaktor 101 mit zwei externen Wärmeübertragern 103, 142 verbindet. Der aus dem Reaktor 101 in den Primärkreislauf 102 geförderte Flüssigkeitsstrom wird auf die beiden Wärmeübertrager aufgeteilt und ist über Stellorgane 108, 140 in den jeweiligen Zulaufleitungen zu den Wärmeübertragern einstellbar. Die in den Wärmeübertragern erhitzten oder abgekühlten Teilströme des Primärkreislaufes werden zum Reaktor zurückgeführt. Aus dem Primärkreislauf 102 wird ein Teil des Flüssigkeitsstroms als flüssiger Reaktoraustrag 123 entnommen. Die Entnahme erfolgt über ein Regelventil 130 in der Entnahmeleitung und über einen Standregler 124 derart, dass ein vorgegebener Flüssigkeitsstand im Reaktor eingehalten wird.

Die Temperatur im Reaktor 101 wird auf einen vorgegebenen Wert eingestellt. Abhängig davon, ob die Reaktion in dem Reaktor endotherm oder exotherm verläuft, muss Wärme dem Reaktor zugeführt oder aus dem Reaktor abgeführt werden. Die beiden Wärmeübertrager leisten hierzu unterschiedliche Beiträge. Während der zweite Wärmeübertrager 142 eine Grundlast des Wärmeaustauschs trägt, wird der erste Wärmeübertrager 103 zur Regelung der Temperatur verwendet. Die Menge des Wärmeträgermediums im Sekundärkreislauf 144 des zweiten Wärmeübertragers 142 ist nicht mengengeregelt, wohingegen die Menge des Wärmeträgermediums im Sekundärkreislauf 104 des ersten Wärmeübertragers 103 über ein Regelventil 109 auf einen vorgegebenen Wert eingestellt wird. Die Menge an Flüssigkeit, die im Primärkreislauf in den zweiten Wärmeübertrager strömt, wird in dem dargestellten Beispiel über einen Durchflussregler 143 und das Regelventil 140 im Zulauf so eingestellt, dass eine Temperatur in einer nachfolgenden Prozessstufe eingehalten wird. Diese Prozessstufe ist in Fig. 1 nicht dargestellt. Die Sollgröße des Durchflusses als Reglerausgang des Temperaturreglers in der nicht dargestellten Prozessstufe ist durch die gestrichelte Linie 141 symbolisiert.

Zur Regelung der Temperatur im Reaktor 101 ist eine Kaskadenregelung vorgesehen, die einen ersten Temperaturregler 105 als Führungsregler und einen zweiten Temperaturregler 113 als Folgeregler umfasst. Dem Führungsregler 105 wird ein Sollwert der Temperatur der Flüssigkeit im Reaktor 101 vorgegeben. Der Istwert der Temperatur im Reaktor 101 wird über einen Temperatursensor erfasst. In Abhängigkeit von der Temperaturdifferenz zwischen dem Istwert und dem Sollwert der Flüssigkeit im Reaktor berechnet der Führungsregler 105 als Ausgangssignal einen Sollwert für die Temperatur der Flüssigkeit, die dem Reaktor als Rücklauf aus den Wärmeübertragern im Primärkreislauf wieder zugeführt wird. Dieser Sollwert wird an den Folgeregler 113 übergeben, der aus der Differenz zwischen dem Istwert und dem Sollwert der Temperatur der rückgeführten Flüssigkeit als Ausgangssignal ein Stellsignal für das Regelventil 108 im Zulauf zum ersten Wärmeübertrager generiert.

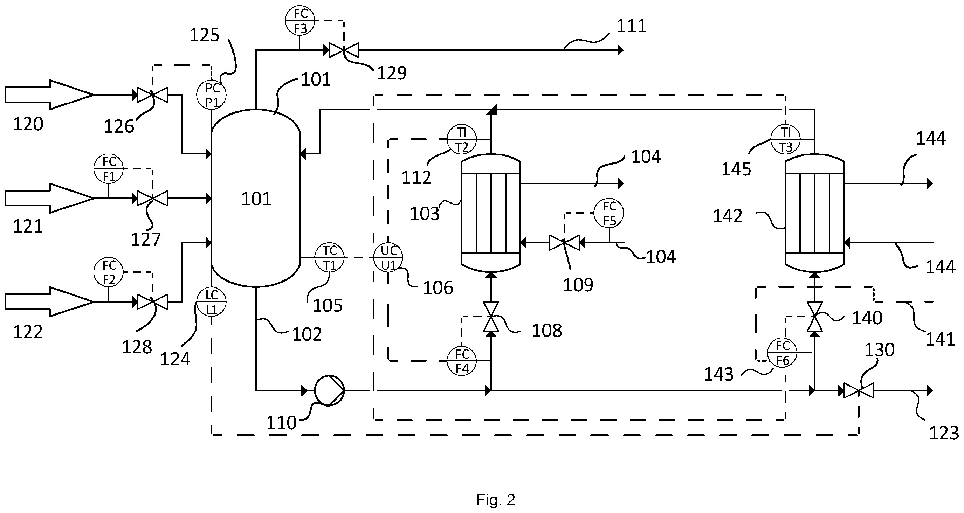

Fig. 2 zeigt schematisch ein Prozessfließbild eines verfahrenstechnischen Prozesses gemäß einer ersten Ausführungsform des erfindungsgemäßen Verfahrens, bei dem die Temperatur in einem Reaktor 101 über externe Wärmeübertrager 103, 142 eingestellt wird. Die Anordnung und Verschaltung der Apparate sowie die Basisregelungen in den Zuführ- und Abführleitungen sind identisch zu dem Verfahren gemäß dem Stand der Technik, sodass diesbezüglich auf die obigen Ausführungen zur Fig. 1 verwiesen wird.

Das erfindungsgemäße Verfahren unterscheidet sich jedoch in der Struktur zur Regelung der Reaktortemperatur von dem Verfahren gemäß dem Stand der Technik. In dem in Fig. 2 dargestellten Beispiel ist zur Regelung der Temperatur im Reaktor 101 eine Kaskadenregelung vorgesehen, die einen Temperaturregler 105 als Führungsregler und einen Wärmestrom-Regler 106 als Folgeregler umfasst. Dem Führungsregler 105 wird ein Sollwert der Temperatur der Flüssigkeit im Reaktor 101 vorgegeben. Der Istwert der Temperatur im Reaktor 101 wird über einen Temperatursensor erfasst. In Abhängigkeit von der Temperaturdifferenz zwischen dem Istwert und dem Sollwert der Flüssigkeit im Reaktor berechnet der Führungsregler 105 als Ausgangssignal einen Sollwert für den der Flüssigkeit im Primärkreislauf 102 durch die Wärmeübertrager 103 und 142 entnommenen oder hinzugefügten Wärmestrom. Dieser Sollwert wird an den Wärmestromfolgeregler 106 übergeben, der aus der Differenz zwischen dem Istwert und dem Sollwert des Wärmestroms als Ausgangssignal ein Stellsignal für das Regelventil 108 im Zulauf zum ersten Wärmeübertrager generiert.

In dem in Fig. 2 dargestellten Beispiel wird der der Flüssigkeit im Primärkreislauf 102 durch die Wärmeübertrager 103 und 142 entnommene oder hinzugefügte Wärmestrom aus einem Anteil des ersten Wärmeübertragers 103 und einem Anteil des zweiten Wärmeübertragers 142 berechnet. Der Anteil des ersten Wärmeübertragers 103 wird ermittelt aus dem durch das Regelventil 108 im Zulauf zum Wärmeübertrager strömenden Flüssigkeitsstrom sowie aus der Temperaturdifferenz zwischen der Temperatur der Flüssigkeit vor und nach dem Wärmeübertrager 103 im Primärkreislauf. Als Temperatur vor dem Wärmeübertrager wird der Istwert der Reaktortemperatur 105 verwendet. Die Temperatur nach dem Wärmeübertrager wird über einen Temperatursensor 112 im Auslass des Wärmeübertragers ermittelt. Analog wird der Anteil des zweiten Wärmeübertragers 142 ermittelt aus dem durch das Regelventil 140 im Zulauf zum Wärmeübertrager strömenden Flüssigkeitsstrom sowie aus der Temperaturdifferenz zwischen der Temperatur der Flüssigkeit vor und nach dem Wärmeübertrager 142 im Primärkreislauf. Als Temperatur vor dem Wärmeübertrager wird der Istwert der Reaktortemperatur 105 verwendet. Die Temperatur nach dem Wärmeübertrager wird über einen Temperatursensor 145 im Auslass des Wärmeübertragers ermittelt.

Die Regelung des Prozesses über den Wärmestrom führt zu einer deutlich besseren Regelgüte im Vergleich zu dem aus dem Stand der Technik bekannten Regelungsverfahren, wie aus den nachfolgend angegebenen Vergleichsbeispiel 1 und Beispiel 1 ersichtlich ist.

In einer Variante der ersten Ausführungsform des erfindungsgemäßen Verfahrens gemäß Fig. 2 werden bei der Berechnung des Stellsignals für das Regelventil 108 Störgrößen berücksichtigt. In dem dargestellten Beispiel handelt es sich bei den Störgrößen um Änderungen der Zusammensetzung und/oder der Menge der Einsatzstoffe. Die Durchflussraten der Einsatzstoffe werden in den Regelventilen 126, 127 und 128 erfasst. Somit sind sowohl die absoluten Mengen als auch deren Verhältnisse zueinander bekannt. Die Messwerte dieser gemessenen Prozessgrößen werden dem Wärmestromregler 106 zugeführt. Die Kenntnis der Störgrößen und deren Berücksichtigung bei der Berechnung des Stellsignals für das Regelventil 108 im Zulauf zum ersten Wärmeübertrager 103 ermöglicht ein noch schnelleres Reagieren auf Regelabweichungen und somit eine noch bessere Regelgüte.

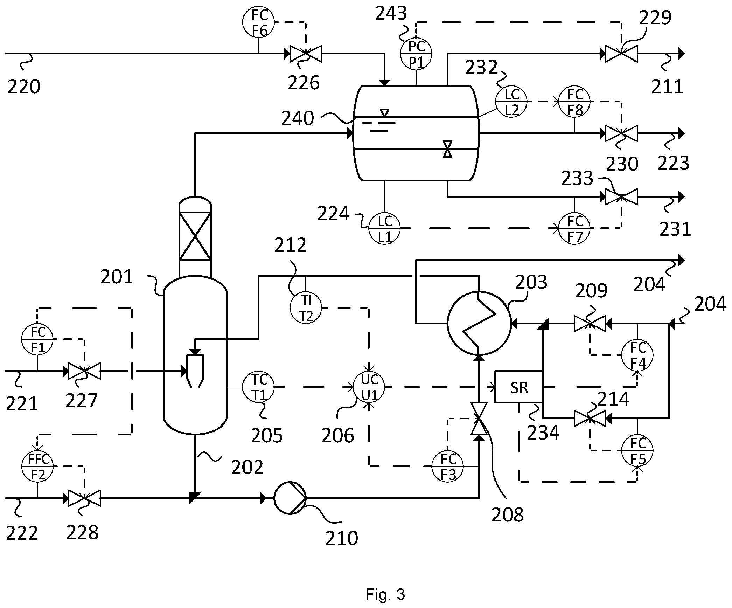

Fig. 3 zeigt schematisch ein Prozessfließbild eines verfahrenstechnischen Prozesses gemäß einer zweiten Ausführungsform des erfindungsgemäßen Verfahrens, bei dem die Temperatur in einem Reaktor 201 als verfahrenstechnischem Apparat über einen externen Wärmeübertrager 203 eingestellt wird. Dem Reaktor 201 wird über eine Leitung ein flüssiger Einsatzstoff 221 zugeführt, der im Reaktor zu einem oder mehreren Produkten umgesetzt wird. Hierbei erfolgt die Zuführung mengengeregelt durch ein Regelventil 227. In einem vorgegebenen Verhältnis zum Flüssigkeitsstrom der Zuführung wird ein weiterer Flüssigkeitsstrom mit Katalysator 222 hinzugefügt, der auch über ein Regelventil 228 mengengeregelt wird.

Das im Reaktor 201 entstehende flüssige Produkt wird zusammen mit der mit Katalysator versetzten Flüssigkeit 222 im oberen Bereich des Reaktors 201 abgezogen und einem Phasenscheider 240 zugeführt.

Um eine mögliche Ausbildung einer explosionsfähigen Atmosphäre im Phasenscheider 240 zu verhindern, wird mengengeregelt ein Inertgas 220 in den Phasenscheider 240 gefördert. Der Druck im Phasenscheider 240 wird über den Abzug von Abgas 232 durch ein Regelventil 229 geregelt, welches vom Gasdruck-Regler 243 im Phasenscheider 240 sein Stellsignal erhält. Hierbei kann das Abgas Inertgas, wie beispielhaft Stickstoff, sowie eventuelle gasförmige bzw. verdampfte Reaktionsprodukte umfassen.

Der Stand der Phasengrenzfläche zwischen den beiden flüssigen Phasen wird über den Abzug des Abwassers 231 durch einen Füllstands-Regler 224 geregelt. Der obere Gesamtstand im Behälter wird über den Produktabzug des flüssigen Produkts 223 geregelt. Hierbei erfolgt die Regelung durch eine Füllstands-Regelung 232, deren Stellsignal das Regelventil 230 steuert.

Ein Teilstrom der sich im Reaktor 201 befindlichen flüssigen Phase wird über eine Pumpe 210 zwangsgefördert in einen Primärkreislauf 202 ausgetragen, der den Reaktor 201 mit einem externen Wärmeübertrager 203 verbindet. Der aus dem Reaktor 201 in den Primärkreislauf 202 geförderte Mengenstrom ist durch das Regelventil 208 einstellbar. Der in dem Wärmeübertrager 203 erhitzte oder abgekühlte Strom im Primärkreislauf wird zum Reaktor 201 zurückgeführt.

Im dargestellten Beispiel wird der der Flüssigkeit im Primärkreislauf 202 durch den Wärmeübertrager 203 entnommene oder hinzugefügte Wärmestrom berechnet. Der entnommene oder hinzugefügte Wärmestrom des Wärmeübertragers 203 wird aus dem durch das Regelventil 208 im Zulauf zum Wärmeübertrager strömenden Flüssigkeitsstrom sowie aus der Temperaturdifferenz zwischen der Temperatur der Flüssigkeit vor und nach dem Wärmeübertrager 203 im Primärkreislauf ermittelt. Als Temperatur vor dem Wärmeübertrager wird der Istwert der Reaktortemperatur 205 verwendet. Die Temperatur nach dem Wärmeübertrager wird über einen Temperatursensor 212 im Auslass des Wärmeübertragers ermittelt.

Im dargestellten Beispiel ist zur Regelung der Temperatur im Reaktor 201 eine Kaskadenregelung vorgesehen, die den Temperaturregler 205 als Führungsregler und einen Wärmestrom-Regler 206 als Folgeregler umfasst. Dem Führungsregler 205 wird ein Sollwert der Temperatur der Flüssigkeit im Reaktor 201 vorgegeben. Der Istwert der Temperatur im Reaktor 201 wird über einen Temperatursensor erfasst. In Abhängigkeit von der Temperaturdifferenz zwischen dem Istwert und dem Sollwert der Flüssigkeit im Reaktor 201 berechnet der Führungsregler 205 als Ausgangssignal einen Sollwert für den der Flüssigkeit im Primärkreislauf 202 durch den Wärmeübertrager 203 entnommenen oder hinzugefügten Wärmestrom. Dieser Sollwert wird an den Wärmestromfolgeregler 206 übergeben, der aus der Differenz zwischen dem Istwert und dem Sollwert des Wärmestroms als Ausgangssignal ein Stellsignal für das Regelventil 208 im Zulauf zum Wärmeübertrager 203 generiert.

Der Regelungsalgorithmus umfasst eine Split-Range-Regelung, bei der zwei Stellsignale berechnet und zwei Mengenströme im Sekundärkreislauf 204 manipuliert werden, wobei ein erster Mengenstrom in Abhängigkeit von einem ersten Stellsignal und ein zweiter Mengenstrom in Abhängigkeit von einem zweiten Stellsignal manipuliert werden.

Hierbei teilt sich der Mengenstrom im Sekundärkreislauf 204 in zwei parallel zueinander angeordneten Mengenströme auf, bevor diese sich wieder vereinen und den Wärmeübertrager 203 durchströmen. Durch die parallele Aufteilung des Mengenstroms kann in jedem der beiden resultierenden Ströme jeweils ein Stellorgan zur Manipulation des jeweiligen Mengenstroms angeordnet werden. In diesem Beispiel sind die beiden Stellorgane mit unterschiedlichen Nennweiten versehen, wodurch signifikant unterschiedliche Durchflussmengen eingestellt werden können. So lässt sich eine präzise Steuerung des Mengenstroms des Wärmeträgermediums gewährleisten, indem das größere der beiden Stellorgane für große Mengenströme ausgelegt ist, und das kleinere der beiden Stellorgane für kleine Mengenströme ausgelegt ist.

Bei der Verwendung von beispielsweise Flusswasser als Wärmeträgermedium im Sekundärkreislauf 204 wird nach dem Durchgang durch den Wärmeübertrager 204 das Flusswasser aus dem System herausgelassen.

Die in Fig. 3 dargestellte zweite Ausführungsform des erfindungsgemäßen Verfahrens eignet sich beispielsweise für Verfahren der Enalisierung von Aldehyden. In derartigen Verfahren werden typischerweise Freistrahlreaktoren 201 zur Umsetzung von Aldehyden mit Wasser in Anwesenheit eines Katalysators eingesetzt, um Enale als Zielprodukte zu erhalten. Bei der Herstellung von Enalen wird Wärme frei, welche über den externen Wärmeübertrager 203 abgeführt wird.

Als flüssiger Einsatzstoff 221 wird dem Reaktor 201 mengengeregelt ein Aldehyd zugeführt. In einem vorgegebenen Verhältnis zum Aldehydstrom wird dem Reaktor 201 ein wässriger Flüssigkeitsstrom 222 zugeführt, der einen Katalysator in wässriger Phase enthält. Das im Reaktor 201 entstehende flüssige Produkt Enal wird zusammen mit der mit Katalysator versetzten Flüssigkeit 222 im oberen Bereich des Reaktors 201 abgezogen und einem Phasenscheider 240 zugeführt. Zur Vermeidung einer möglichen Ausbildung einer explosionsfähigen Atmosphäre wird dem Phasenscheider 240 Stickstoff als Inertgas 220 zugeführt. Im Phasenscheider 240 wird die den Katalysator enthaltende flüssige Phase über die Leitung 231 entnommen. Das Zielprodukt Enal wird in der organischen Phase über die Leitung 223 entnommen.

Ein Teilstrom der sich im Reaktor 201 befindlichen flüssigen Phase wird als Umwälzstrom über eine Pumpe 210 zwangsgefördert in einen Primärkreislauf 202 ausgetragen. Dabei wird dieser Flüssigkeitsstrom so gewählt, dass sich im Reaktor 201 eine intensive Durchmischung der organischen und der wässrigen Phase einstellt. Dies resultiert aus einem Leistungseintrag, der durch die Umwälzung in die Flüssigkeit eingebracht wird. Der Leistungseintrag durch den Flüssigkeitsstrom in den Reaktor 201 wird somit einstellbar.

Bei dieser Ausführungsform wird vorteilhafterweise der Wärmestrom durch den Mengenstrom des Wärmeträgermediums im Sekundärkreislauf 204 geregelt.

Zur Regelung der diversen Regelkreise werden vorzugsweise PID-Regler mit auf den gewünschten stationären Zustand optimierten Reglerparametern eingesetzt. Die Berechnung des Istwertes des im Wärmeübertrager 203 entnommenen Wärmestroms erfolgt mit dem Messwert der Temperatur $T_{in}$ im Reaktor 201, dem Messwert der Temperatur $T_{out,1}$ 212 nach dem Wärmeübertrager 203, dem Messwert des Massenstroms $\dot{m}_1$ durch das Regelventil 208 im Zulauf zum Wärmeübertrager 203 und dem Parameter der Wärmekapazität $c_l$ für das Medium des Flüssigkeitsstroms nach der Formel $\dot{Q}_1 = c_l \dot{m}_1 (T_{in} - T_{out,1})$.

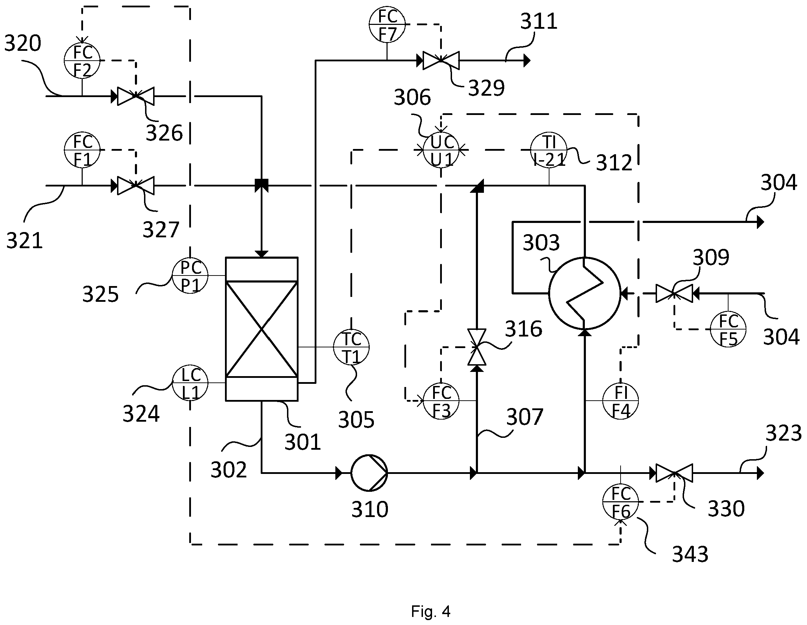

Fig. 4 zeigt schematisch ein Prozessfließbild eines verfahrenstechnischen Prozesses gemäß einer dritten Ausführungsform des erfindungsgemäßen Verfahrens, bei dem die Temperatur in einem Reaktor 301 als verfahrenstechnischem Apparat über einen externen Wärmeübertrager 303 eingestellt wird. Dem Reaktor 301 wird über eine Leitung ein flüssiger Einsatzstoff 321 zugeführt, der im Reaktor zu einem oder mehreren Produkten umgesetzt wird. Hierbei erfolgt die Zuführung mengengeregelt durch ein Regelventil 327.

Ein Gasstrom 320 wird dem Reaktor 301 hinzugefügt, der über ein Regelventil 326 mengengeregelt wird. Hierbei erhält das Regelventil 326 sein Stellsignal von einem Druckregler 325, der den Druck im Reaktor regelt. Über einen gasförmigen Austrag 311 werden gasförmige Nebenprodukte aus dem Reaktor durch ein Regelventil 329 mengengeregelt entfernt.

Das im Reaktor 301 entstehende flüssige Produkt wird durch eine Umwälzpumpe 310 aus dem Reaktor 301 in einen Primärkreislauf 302 gefördert, der den Reaktor 301 mit einem externen Wärmeübertrager 303 verbindet.

Der Flüssigkeitsstrom durch den Wärmeübertrager 303 ist indirekt auch durch ein Regelventil 316, welches sich in einem Bypass 307 im Primärkreislauf befindet, einstellbar. Hierbei ist der Bypass im Primärkreislauf parallel zu dem Wärmeübertrager 303 geschaltet.

Der in dem Wärmeübertrager 303 erhitzte oder abgekühlte Strom sowie der Strom durch den Bypass 307 im Primärkreislauf werden zum Reaktor 301 zurückgeführt.

Aus dem Primärkreislauf 302 wird ein Teil des Flüssigkeitsstroms als flüssiger Reaktoraustrag 323 entnommen. Die Entnahme erfolgt über einen Durchflussregler 343 und ein Regelventil 330 in der Entnahmeleitung und über einen Standregler 324 derart, dass ein vorgegebener Flüssigkeitsstand im Reaktor eingehalten wird.

Im dargestellten Beispiel wird der der Flüssigkeit im Primärkreislauf 302 durch den Wärmeübertrager 303 entnommene oder hinzugefügte Wärmestrom berechnet aus dem durch den Wärmeübertrager 303 strömenden Flüssigkeitsstrom sowie aus der Temperaturdifferenz zwischen der Temperatur der Flüssigkeit vor und nach dem Wärmeübertrager 303 im Primärkreislauf. Als Temperatur vor dem Wärmeübertrager wird der Istwert der Reaktortemperatur 305 verwendet. Die Temperatur nach dem Wärmeübertrager wird über einen Temperatursensor 312 im Auslass des Wärmeübertragers ermittelt.

In diesem erfindungsgemäßen Beispiel ist zur Regelung der Temperatur im Reaktor 301 eine Kaskadenregelung vorgesehen, die den Temperaturregler 305 als Führungsregler und einen Wärmestromfolgeregler 306 umfasst. Dem Temperaturregler 305 wird ein Sollwert der Temperatur der Flüssigkeit im Reaktor 301 vorgegeben. Der Istwert der Temperatur im Reaktor 301 wird über einen Temperatursensor 305 erfasst. In Abhängigkeit von der Temperaturdifferenz zwischen dem Istwert und dem Sollwert der Flüssigkeit im Reaktor 301 berechnet der Wärmestromfolgeregler 306 als Ausgangssignal einen Sollwert für den der Flüssigkeit im Primärkreislauf 302 durch den Wärmeübertrager 303 entnommenen oder hinzugefügten Wärmestrom. Dieser Sollwert wird an den Wärmestromfolgeregler 306 übergeben, der aus der Differenz zwischen dem Istwert und dem Sollwert des Wärmestroms als Ausgangssignal ein Stellsignal für das Regelventil 316 im Bypass 307 generiert.

Die Menge des Wärmeträgermediums im Sekundärkreislauf 304 des Wärmeübertragers 303 ist durch ein Regelventil 309 mengengeregelt und wird meist auf einen festen Wert eingestellt. Das Wärmeträgermedium im Sekundärkreislauf 304 wird entsprechend den Anforderungen an die erforderliche Kühl- oder Heizleistung gewählt. Im Falle einer Kühlung wird vorzugsweise Flusswasser oder gekühltes Wasser als Wärmeträgermedium verwendet.

Die in Fig. 4 dargestellte dritte Ausführungsform des erfindungsgemäßen Verfahrens eignet sich beispielsweise für Verfahren zur Hydrierung von Alkenen und Aldehyden, Selektivhydrierungen wie die Selektivhydrierung von Butadien und Veretherungen. In derartigen Verfahren werden typischerweise Festbettreaktoren 301 zur Umsetzung der Ausgangsstoffe zu den Zielprodukten eingesetzt.

Im Beispiel einer Hydrierung wird dem Reaktor 301 der zu hydrierende flüssige Einsatzstoff über die Leitung 321 mengengeregelt zugeführt. Wasserstoff wird dem Reaktor 301 gasförmig über die Leitung 320 druckgeregelt zugeführt. Über einen gasförmigen Austrag 311 werden gasförmige Nebenprodukte aus dem Reaktor durch ein Regelventil 329 mengengeregelt entfernt. Das im Reaktor 301 entstehende flüssige Produkt, beispielsweise ein Butanol, wird aus dem Primärkreislauf über die Leitung 323 mengengeregelt entnommen.

Zur Regelung der diversen Regelkreise werden vorzugsweise PID-Regler mit auf den gewünschten stationären Zustand optimierten Reglerparametern eingesetzt. Die Berechnung des Istwertes des im Wärmeübertrager 303 entnommenen Wärmestroms erfolgt mit dem Messwert der Temperatur $T_{in}$ im Reaktor 301, dem Messwert der Temperatur $T_{out,1}$ 312 nach dem Wärmeübertrager 303, dem Messwert des Massenstroms $\dot{m}_1$ im Zulauf zum Wärmeübertrager 303 und dem Parameter der Wärmekapazität $c_l$ für das Medium des Flüssigkeitsstroms nach der Formel $\dot{Q}_1 = c_l \dot{m}_1 (T_{in} - T_{out,1})$.

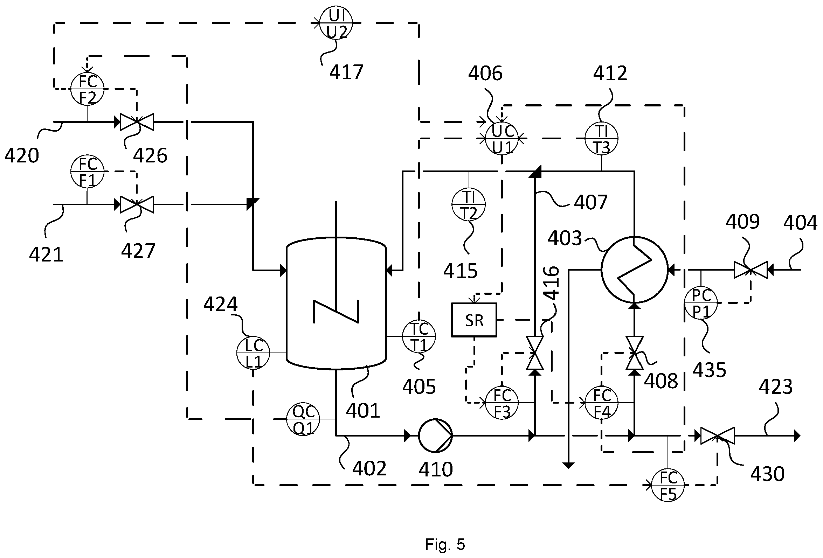

Fig. 5 zeigt schematisch ein Prozessfließbild eines verfahrenstechnischen Prozesses gemäß einer vierten Ausführungsform des erfindungsgemäßen Verfahrens, bei dem die Temperatur in einem Reaktor 401 über einen externen Wärmeübertrager 403 eingestellt wird. Dem Reaktor 401 wird über eine Leitung ein erster flüssiger Einsatzstoff 421 zugeführt, der im Reaktor zu einem oder mehreren Produkten umgesetzt wird. Hierbei erfolgt die Zuführung mengengeregelt durch ein Regelventil 427. Ein zweiter flüssiger Einsatzstoff 420 wird dem Reaktor 401 hinzugefügt, der über ein Regelventil 426 auf Basis einer gemessenen Stoffkonzentration (Q1) im Flüssigkeitsstrom des Primärkreislaufes 402 geregelt wird. Die Stoffkonzentration kann auch durch eine Ersatzgröße, beispielsweise Leitfähigkeit, Dichte, Viskosität oder Schallgeschwindigkeit, ermittelt werden, insofern die Ersatzgröße einen Rückschluss auf die Stoffkonzentration ermöglicht.

Das im Reaktor 401 entstehende flüssige Produkt wird durch eine Umwälzpumpe 410 aus dem Reaktor 401 in einen Primärkreislauf 402 gefördert, der den Reaktor 401 mit einem externen Wärmeübertrager 403 verbindet. Der Mengenstrom durch den Wärmeübertrager 403 ist indirekt durch ein Regelventil 416 in einem Bypass 407 und direkt durch ein Regelventil 408 vor dem Wärmeübertrager 403 einstellbar.

Der in dem Wärmeübertrager 403 erhitzte oder abgekühlte Strom sowie der Strom durch den Bypass 407 im Primärkreislauf werden zum Reaktor 401 zurückgeführt.

Aus dem Primärkreislauf 402 wird ein Teil des Flüssigkeitsstroms als flüssiger Reaktoraustrag 423 entnommen. Die Entnahme erfolgt über einen Durchflussregler und ein Regelventil 430 in der Entnahmeleitung und über einen Standregler 424 derart, dass ein vorgegebener Flüssigkeitsstand im Reaktor eingehalten wird.

In diesem Beispiel wird der der Flüssigkeit im Primärkreislauf 402 durch den Wärmeübertrager 403 entnommene oder hinzugefügte Wärmestrom berechnet aus dem durch den Wärmeübertrager 403 strömenden Flüssigkeitsstrom sowie aus der Temperaturdifferenz zwischen der Temperatur der Flüssigkeit vor und nach dem Wärmeübertrager 403 im Primärkreislauf. Als Temperatur vor dem Wärmeübertrager wird der Istwert der Reaktortemperatur 405 verwendet. Die Temperatur nach dem Wärmeübertrager wird über einen Temperatursensor 412 im Auslass des Wärmeübertragers ermittelt.

In diesem erfindungsgemäßen Beispiel ist zur Regelung der Temperatur im Reaktor 401 eine Kaskadenregelung vorgesehen, die den Temperaturregler 405 als Führungsregler und einen Wärmestrom-Regler 406 als Folgeregler umfasst. Dem Führungsregler 405 wird ein Sollwert der Temperatur der Flüssigkeit im Reaktor 401 vorgegeben. Der Istwert der Temperatur im Reaktor 401 wird über einen Temperatursensor 405 erfasst. In Abhängigkeit von der Temperaturdifferenz zwischen dem Istwert und dem Sollwert der Flüssigkeit im Reaktor 401 berechnet der Führungsregler 405 als Ausgangssignal einen Sollwert für den der Flüssigkeit im Primärkreislauf 402 durch den Wärmeübertrager 403 entnommenen oder hinzugefügten Wärmestrom. Dieser Sollwert wird an den Wärmestromfolgeregler 406 übergeben, der aus der Differenz zwischen dem Istwert und dem Sollwert des Wärmestroms Stellsignale berechnet. Der Regelungsalgorithmus des Folgereglers 406 ist als Split-Range-Regelung ausgeführt, bei der zwei Stellsignale berechnet werden. In Abhängigkeit von einem ersten Stellsignal wird der Flüssigkeitsstrom im Bypass 407 über das Regelventil 416 manipuliert. In Abhängigkeit von einem zweiten Stellsignal wird der Flüssigkeitsstrom im Primärkreislauf 402 durch das Regelventil 408 im Zulauf des Wärmeübertragers 403 manipuliert. Je nach einzustellenden Flüssigkeitsströmen können die beiden Regelventile 408, 416 unabhängig voneinander zwischen ihren Geschlossenstellungen und ihren Offenstellungen eingestellt werden.

In dem in Fig. 5 dargestellten Beispiel berücksichtigt der Regelungsalgorithmus bei der Berechnung des Stellsignals auch Störgrößen. Eine Störgröße, die in dem beispielhaften Prozess bekannt ist, ist der Einfluss der zugeführten Menge an Einsatzstoff auf die Wärmeentwicklung im Reaktor. In Abhängigkeit davon, ob die Reaktion exotherm oder endotherm verläuft, bewirkt die Zufuhr des zweiten Einsatzstoffes 420 zum ersten Einsatzstoff 421 die Zunahme oder Abnahme der Temperatur im Reaktor 401. Die aufgrund der zugeführten Menge an zweitem Einsatzstoff 420 positive oder negative Wärmemenge wird berechnet und dem Folgeregler 406 als Störgröße aufgeschaltet. Die Berechnung der Wärmemenge kann innerhalb des Folgereglers erfolgen oder, wie in Fig. 5 dargestellt, in einem separaten Modellbaustein 417. Durch die Berücksichtigung der Störgröße ist der Regelungsalgorithmus in der Lage, auf die zu erwartende Veränderung der Temperatur im Reaktor 401 vorausschauend zu reagieren, noch bevor die Veränderung über den Temperatursensor 405 erkennbar ist. Dadurch können Laständerungen, Schwankungen oder Sollwertänderungen im Prozess schneller und besser geregelt werden, was zu einer verbesserten Regelgüte führt.

Der Mengenstrom des Wärmeträgermediums im Sekundärkreislauf 404 des Wärmeübertragers 403 wird durch ein Regelventil 409 und einen Druckregler 435 so eingestellt, dass ein vorgegebener Druck im Zulauf des Wärmeträgermediums zum Wärmeübertrager gehalten wird. Dies ist insbesondere dann vorteilhaft, wenn Heizdampf als Wärmeträgermedium verwendet wird. Das Wärmeträgermedium im Sekundärkreislauf 404 wird entsprechend den Anforderungen an die erforderliche Kühl- oder Heizleistung gewählt. Im Falle einer Heizung wird bevorzugt Heizdampf als Wärmeträgermedium im Sekundärkreislauf 404 verwendet.

Die in Fig. 5 dargestellte vierte Ausführungsform des erfindungsgemäßen Verfahrens eignet sich beispielsweise für Verfahren, bei denen der verfahrenstechnische Apparat 401 ein Rührbehälter ist, in dem verschiedene Substanzen miteinander vermischt werden oder eine Substanz in einer anderen Substanz gelöst werden soll.

Im Beispiel der Herstellung einer wässrigen Lösung von Ammoniumnitrat wird dem Rührbehälter 401 über die Leitung 421 Wasser mengengeregelt zugeführt. Das zu lösende Ammoniumnitrat wird dem Rührbehälter über die Leitung 420 zugeführt. Die Zuführung erfolgt mengengeregelt, wobei der Durchflussregler (FC) als Folgeregler seinen Sollwert von einem Führungsregler (QC) erhält, der als Qualitätsregler eine vorgegebene Konzentration einer Komponente oder eine vorgegebene Ersatzgröße, die einen Rückschluss auf die Konzentration einer Komponente zulässt, im in den Primärkreislauf 402 abgezogenen Flüssigkeitsstrom regelt.

Der Qualitätsregler sorgt für eine konstante Zusammensetzung der hergestellten Lösung. Beispielsweise dient die Leitfähigkeit der abgeführten Lösung als leicht zugängliches Qualitätsmaß. Der Stand im Rührbehälter 401 wird über einen Füllstand-Regler 424, der den Abzug der hergestellten Lösung 423 durch ein Regelventil 430 manipuliert, geregelt. Der Prozess ist endotherm, sodass die im Rührbehälter 401 entzogene Wärme durch den Wärmeübertrager 403 wieder zugeführt werden muss. Dazu wird Heizdampf verwendet, dessen Druck in der Zuführleitung 404 über einen Druckregler 435, welcher das Regelventil 409 ansteuert, geregelt wird.

Zur Regelung der diversen Regelkreise werden vorzugsweise PID-Regler mit auf den gewünschten stationären Zustand optimierten Reglerparametern eingesetzt. Die Berechnung des Istwertes des im Wärmeübertrager 403 entnommenen Wärmestroms erfolgt mit dem Messwert der Temperatur $T_{in}$ im Reaktor 401, dem Messwert der Temperatur $T_{out,1}$ 412 nach dem Wärmeübertrager 403, dem Messwert des Massenstroms $\dot{m}_1$ im Zulauf zum Wärmeübertrager 303 und dem Parameter der Wärmekapazität $c_l$ für das Medium des Flüssigkeitsstroms nach der Formel $\dot{Q}_1 = c_l \dot{m}_1 (T_{in} - T_{out,1})$.

Als Störgrößenaufschaltung wird der zu erwartende zuzuführende Wärmemengenstrom auf Basis des Ammoniumnitrats 420 durch eine Wärmemengenberechnung 417 erfasst und dem Wärmemengenregler 406 als Störgröße aufgeschaltet.

Beispiele:

Vergleichsbeispiel 1

Die Hydroformylierung von Propen wird großtechnisch in einem Blasensäulenreaktor mittels eines Rhodium-Triphenylphosphin-Komplexes als Katalysator durchgeführt. Bei der Reaktion wird Wärme freigesetzt, welche aus dem Reaktor abzuführen ist. Das verfahrenstechnische Prozessfließbild eines solchen Prozesses gemäß dem Stand der Technik ist in Fig. 1 angegeben.

Dem Blasensäulenreaktor 101 werden Wasserstoff und Kohlenmonoxid gemeinsam als sogenanntes Synthesegas über die Leitung 120 als gasförmiger Einsatzstoff zugeführt. Über die Leitung 121 wird Propen flüssig als weiterer Einsatzstoff mengengeregelt zugeführt. Um bei den vorgegebenen Reaktionsbedingungen eine flüssige Phase zu erhalten, wird ein flüssiges Lösungsmittel, das einen Katalysator enthält, über die Leitung 122 mengengeregelt zugegeben. Die bei den Reaktionsbedingungen gasförmigen Einsatzstoffe lösen sich in dem Lösungsmittel und reagieren in Gegenwart des homogenen Katalysators zu den gewünschten Reaktionsprodukten. Nicht umgesetzte gasförmige Einsatzstoffe, insbesondere Wasserstoff und Kohlenmonoxid, werden über den gasförmigen Reaktoraustrag 111 entfernt. Das Zielprodukt der Reaktion wird über den flüssigen Reaktoraustrag 123 aus dem Primärkreislauf 102 entnommen. Da die Reaktion exotherm ist, wird der flüssige Reaktorinhalt gekühlt, um die Reaktortemperatur auf einem gewünschten konstanten Wert zu halten. Dies erfolgt durch die Kühlung des Flüssigkeitsstromes im Primärkreislauf 102 in den Wärmeübertragern 103 und 142, beispielsweise mit Flusswasser als Wärmeträgermedium in den Sekundärkreisläufen. Zur Regelung der diversen Regelkreise werden PID-Regler mit auf den gewünschten stationären Zustand optimierten Reglerparametern eingesetzt.

Beispiel 1

Derselbe Prozess wie in Vergleichsbeispiel 1 wird mit einer erfindungsgemäßen Reglerkonfiguration wie in Fig. 2 angegeben betrieben. Auch in diesem Fall werden zur Regelung der diversen Regelkreise PID-Regler mit auf den gewünschten stationären Zustand optimierten Reglerparametern eingesetzt. Die optimalen Regler-Parameter wurden über geeignete Einstellregeln bestimmt, so wie sie beispielsweise in folgenden Fachliteraturquellen erläutert werden:

- Otto Föllinger, Regelungstechnik, 8. Auflage, 1994, Hüthig Verlag
- Heinz Unbehauen, Regelungstechnik I, 13. Auflage, 2005, Vieweg Verlag
- S. Skogestad und Ian Postlethwaite, Multivariable Feedback Control, 2005, Wiley Verlag
- The systematic design of PID controllers using Nyquist stability (DOI: 10.23919/ECC.2001.7075961)

In dem in Fig. 2 dargestellten Beispiel ist zur Regelung der Temperatur im Reaktor 101 eine Kaskadenregelung vorgesehen, die einen Temperaturregler 105 als Führungsregler und einen Wärmestrom-Regler 106 als Folgeregler umfasst. Dem Führungsregler 105 wird ein Sollwert der Temperatur der Flüssigkeit im Reaktor 101 vorgegeben. Der Istwert der Temperatur im Reaktor 101 wird über einen Temperatursensor erfasst. In Abhängigkeit von der Temperaturdifferenz zwischen dem Istwert und dem Sollwert der Flüssigkeit im Reaktor berechnet der Führungsregler 105 als Ausgangssignal einen Sollwert für den der Flüssigkeit im Primärkreislauf 102 durch die Wärmeübertrager 103 und 142 entnommenen oder hinzugefügten Wärmestrom. Dieser Sollwert wird an den Wärmestromfolgeregler 106 übergeben, der aus der Differenz zwischen dem Istwert und dem Sollwert des Wärmestroms als Ausgangssignal ein Stellsignal für das Regelventil 108 im Zulauf zum ersten Wärmeübertrager generiert.

Die Berechnung des Istwertes des im ersten Wärmeübertrager 103 entnommenen Wärmestroms erfolgt mit dem Messwert der Temperatur $T_{in}$ im Reaktor 101, dem Messwert der Temperatur $T_{out,1}$ nach dem ersten Wärmeübertrager 103, dem Messwert des Massenstroms $\dot{m}_1$ durch das Regelventil 108 im Zulauf zum ersten Wärmeübertrager 103 und dem Parameter der Wärmekapazität $c_l$ für das Medium des Flüssigkeitsstroms nach der Formel $\dot{Q}_1 = c_l \dot{m}_1(T_{in} - T_{out,1})$. Die Berechnung des Istwertes des im zweiten Wärmeübertrager 142 entnommenen Wärmestroms erfolgt mit dem Messwert der Temperatur $T_{in}$ im Reaktor 101, dem Messwert der Temperatur $T_{out,2}$ nach dem zweiten Wärmeübertrager 142, dem Messwert des Massenstroms $\dot{m}_2$ durch das Regelventil 140 im Zulauf zum zweiten Wärmeübertrager 142 und dem Parameter der Wärmekapazität $c_l$ für das Medium des Flüssigkeitsstroms nach der

$$\text{Formel } \dot{Q}_2 = c_l \dot{m}_2 \left( T_{in} - T_{out,2} \right).$$

Der Prozess gemäß Stand der Technik und der erfindungsgemäße Prozess wurden in dem Simulationstool "Aspen Plus Dynamics" der Firma AspenTech (20 Crosby Drive, Bedford, MA 01730, U.S.A., www.aspentech.com) simuliert. Dem Simulationsmodell lagen die folgenden Annahmen und Parameter zugrunde:
Der Gesamtdruck im Reaktor 101 ist durch die Summe der Partialdrücke von Wasserstoff, Kohlenstoffmonoxid, Lösungsmittel und Propen gegeben. Es werden keine anderen Komponenten in der Gasphase berücksichtigt Der Katalysator-Molanteil $x_{Cat}$ bleibt konstant während der Simulation. Zudem wird keine Wärme zwischen dem Reaktor 101 oder den Leitungen und der Umgebung ausgetauscht, sodass adiabatische Bedingungen angenommen werden. Im Folgenden werden die Randbedingungen der Simulation aufgelistet:

- Der Wärmestromdurchgangs-Koeffizient der externen Wärmeübertrager beträgt konstant $U = 850 \frac{W}{m^2\,K}$.
- Die Eingangstemperatur des Wärmeträgermediums im Sekundärkreislauf ist $T_{in,C}$ = 48 °C.
- Die Ausgangstemperatur des Wärmeträgermediums im Sekundärkreislauf des ersten Wärmeübertragers 103 bei normaler Last beträgt $T_{out,C,nom}$ = 73 °C.
- Der durch den ersten Wärmeübertrager 103 entnommene Wärmestrom bei Nennlast ist $\dot{Q}_{HE,nom}$ = 21600 kW.
- Der durch den zweiten Wärmeübertrager 142 entnommene Wärmestrom bei Simulation des Störverhaltens ist $\dot{Q}_{HE1,nom}$ = 2160 kW, wobei der totale stationäre Wärmestrom durch beide Wärmeübertrager $\dot{Q}_{TOT,nom} = Q_{Reak,nom}$ = 21600 kW beträgt. D.h. die Wärmefreisetzung bleibt von der Störung unberührt.
- Der Flüssigkeitsstrom zum ersten Wärmeübertrager bei normaler Last beträgt

$$\dot{m}_{HE,PF} = 1816 \frac{t}{h}$$

- Der Flüssigkeitsstrom zum zweiten Wärmeübertrager bei Simulation des Störverhaltens beträgt

$$\dot{m}_{HE1,PF} = 181,6 \; \frac{t}{h}$$

- Die Wärmestromgleichung lautet: $\dot{Q}_{HE} = U * A * \Delta T_m = \dot{m}_{PF,HE} \, c_{PF,l} \, (T_{in,PF} - T_{out,PF}) = \dot{m}_{HE,C} \, c_{C,l} \, (T_{out,C} - T_{in,C})$
- Die einzige Wärmesenke sind die beiden Wärmeübertrager.
- Die Temperatur des Flüssigkeitsstroms $T_{in,PF}$ ist gleich der Reaktortemperatur $T_R$.
- Beide Wärmeübertrager arbeiten im Gegenstromprinzip: $T_{out,PF} - T_{1,PF}$, $T_{in,PF} = T_{2,PF}$, $T_{in,C} = T_{1,C}$ und $T_{out,C} = T_{2,C}$
- Die mittlere logarithmische Temperaturdifferenz ist gegeben durch:

$$\Delta T_m = \frac{\Delta T_1 - \Delta T_2}{ln\left(\frac{\Delta T_1}{\Delta T_2}\right)} = \frac{\left(T_{1,PF} - T_{1,C}\right) - \left(T_{2,PF} - T_{2,C}\right)}{ln\left(\frac{\left(T_{1,PF} - T_{1,C}\right)}{\left(T_{2,PF} - T_{2,C}\right)}\right)}$$

- Die Reaktortemperatur bei normaler Last beträgt $T_R$ = 95 °C.

$$\dot{r}_{nom} = 5,6 \; \frac{mol}{l \, h}$$

- Die Reaktionsrate bei normaler Last beträgt
- Der Gesamtdruck im Reaktor beträgt p = 10 *bar.*
- Der Partialdruck von Kohlenstoffmonoxid bei normaler Last ist $P_{CO,nom}$ = 3 *bar.*
- Bei normaler Last ist das Verhältnis zwischen Wasserstoff und Kohlenstoffmonoxid im Strom 120 so zu wählen, dass der Partialdruck von Wasserstoff $P_{H2,nom}$ gleich dem von Kohlenstoffmonoxid $p_{CO,nom}$ ist.
- Die Reaktionsrate wird berechnet durch

$$\dot{r} = k_{R,0} \, e^{-\frac{E_A}{R \, T_R}} \, c_{Propene} \, c_{Cat} \, \frac{c_{CO}}{1 + K_{CO} \, c_{CO}{}^2} \, \frac{c_{H_2}}{1 + K_{H_2} \, c_{H_2}}$$

Hierbei sind $c_{CO}$, $c_{H_2}$, $C_{Propene}$ und $c_{Cat}$ Konzentrationen der individuellen Komponenten in mol/l, $E_A$ is die Aktivierungsenergie und $k_{R,0}$ ist der präexponentielle Faktor.
- Die Enthalpie der Reaktion ist berechnet durch das in dem Simulationstool vorgegebenen Enthalpiemodell (Aspen Properties) für die flüssige Phase und die Anwendung des Satz von Hess.

Die Parameter $K_{CO}$, $K_{H_2}$, $E_A$ und $k_{R,0}$ der Reaktionskinetik wurden angepasst, um die negative Korrelation der Reaktionsrate mit zunehmendem Kohlenstoffmonoxid-Partialdruck abzubilden. Die verwendeten Parameter sind:

$$K_{CO} = 45000 \, \frac{dm^6}{mol^2}, \; K_{H_2} = 500 \, \frac{dm^3}{mol}, \; E_A = 60000 \, \frac{J}{mol}$$

und

$$k_{R,0} \, c_{Cat} = 4.2465 \; 10^{15} \, \frac{mol^2}{dm^3 \, h}.$$

Da die Katalysatorkonzentration $c_{Cat}$ unbekannt, aber konstant, ist, wurde diese mit dem präexponentiellen Faktor $k_{R,0}$ zusammengeführt. Dies ist gleichbedeutend mit dem Zahlenwert von 1 für die Katalysatorkonzentration $c_{Cat}$. Die Reaktionsrate ist eine Funktion der Konzentrationen ohne Berücksichtigung der Partialdrücke.
Die Korrelation zwischen den Partialdrücken und den Konzentrationen kann durch die bekannten Löslichkeiten der einzelnen Gase in der flüssigen Phase ermittelt werden. Die Löslichkeit der Gase hängt von dem Lösemittel ab, in welchem die Reaktion abläuft. Das Lösemittel ist im Allgemeinen eine Mischung aus Rhodium Katalysator, Ligand und n-Butyraldehyd, Isobutyraldehyd, Dimeren, Trimeren und höheren Oligomeren von Butyraldehyd. Dies bedeutet, dass das Lösemittel zu großen Teilen aus schwersiedenden Komponenten besteht, die durch die Nebenreaktionen der Hydroformulierung entstehen. Zur Vereinfachung wurde angenommen, dass die Mischung (inkl. Katalysator und Ligand) durch die Simulation einer einzigen repräsentativen Komponente beschrieben werden kann. Die Komponente Texanol wurde als Lösemittel ausgewählt, da ihre Parameter in Aspen Properties zur Verfügung stehen und diese mit der beschriebenen realen Mischung gut übereinstimmen.

Es wurde die physikalische Zustandsgleichung "Predictive Soave Redlich Kwong" (PSRK) verwendet, um die Löslichkeit und die anderen Eigenschaften zu ermitteln und die dynamischen Simulationen durchzuführen. Die Löslichkeit ist eine Funktion von Druck, Temperatur und den Komponenten der flüssigen Phase, welche durch die Zustandsgleichung berechnet wird.

Um den Reaktor 101 zu dimensionieren, muss ein Umsatz für den einfachen Durchgang von Synthesegas 120 und Propen 121 vorgegeben werden. Der Umsatz im einfachen Durch-gang $X_{SP,Propene}$ ist hier definiert als der Quotient aus dem Mengenstrom an Butanal $\dot{n}_{Butanal,Rework}$, der der Aufarbeitungssektion zugeführt wird, zu dem Mengenstrom an Propen $\dot{n}_{Propene,Feed}$, der dem Reaktor 101 zugeführt wird. Diese Definition wurde gewählt, da im Abgas ein erheblicher Teil des nicht umgesetzten Propens enthalten ist und damit die Summe über mehrere Ströme zu bilden wäre, wenn das nicht umgesetzte Propen zu ermitteln wäre. Der Umsatz für den einfachen Durchgang des Synthesegases 120 wird so eingestellt, dass der Molanteil von Kohlenstoffmonoxid im gasförmigen Reaktionsaustrag $y_{CO,Vent,nom}$ in Richtung Aufarbeitung $0.3 \frac{mol}{mol}$ beträgt.

Die Synthesegaszusammensetzung im Zustrom ist so eingestellt, dass der Molanteil von Wasserstoff im gasförmigen Reaktionsaustrag bei normaler Last gleich dem Molanteil von Kohlenstoffmonoxid im gasförmigen Reaktionsaustrag bei nominaler Last ist. Der Partialdruck von Wasserstoff und Kohlenstoffmonoxid ist ca. 3 bar und die Hydroformulierungsreaktion ist im Bereich von negativer Korrelation zwischen dem Partialdruck von Kohlenstoffmonoxid und der Reaktionsrate.

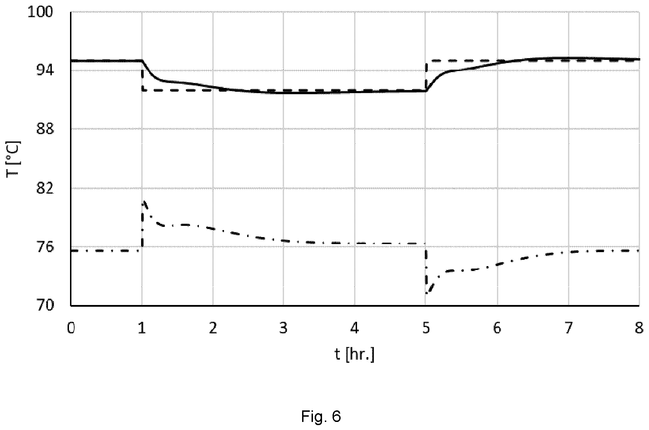

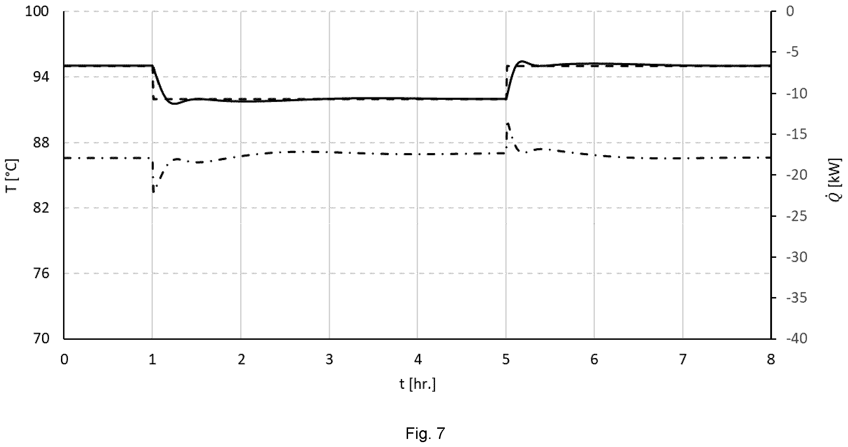

Ein Vergleich der Simulationsergebnisse zwischen Vergleichsbeispiel 1 und Beispiel 1 für das Führungsverhalten des Reglers bei einer Sollwertänderung der Reaktortemperatur von 95°C auf 92°C und zurück auf 95°C wird in Fig. 6 und Fig. 7 dargestellt. Hierbei wird für beide Beispiele kein zweiter Wärmeübertrager 142 hinzugeschaltet.

In dem Vergleichsbeispiel 1 wird eine Temperatur-Temperatur-Kaskade verwendet, um die Temperatur im Reaktor zu regeln. Hingegen wird im erfindungsgemäßen Beispiel 1 eine Temperatur-Wärmestrom-Kaskade verwendet, um die Temperatur im Reaktor zu regeln. Zum Einpendeln des Verfahrens in den Simulationen werden in beiden Fällen für die ersten 60 Minuten die Verfahrensrandbedingungen, wie beispielsweise die Reaktortemperatur oder die Eduktströme, konstant gehalten. Hierbei wird "Aspen Plus Dynamics" verwendet. Nach einer Stunde simulierter Zeit wird der Sollwert der Reaktortemperatur instantan von 95°C auf 92°C abgesenkt. Nach weiteren vier Stunden simulierter Zeit wird der Sollwert der Reaktortemperatur wieder auf seinen ursprünglichen Wert von 95°C zurückgesetzt. In Fig. 6 und Fig. 7 zeigt jeweils die gestrichelte Linie den Sollwert der Reaktortemperatur, die durchgezogene Linie den Istwert der Reaktortemperatur und die strich-gepunktete Linie das Stellsignal des Führungsreglers 105. Im Fall des Vergleichsbeispiels ist dies eine Soll-Temperatur für den Folgeregler, im Fall des erfindungsgemäßen Beispiels ein Soll-Wärmestrom für den Folgeregler. Wie aus einem Vergleich der Kurven der Istwerte der Reaktortemperatur klar zu erkennen ist, wird durch das erfindungsgemäße Verfahren der Sollwert signifikant schneller erreicht als durch das Verfahren gemäß dem Stand der Technik. Die Regelgüte ist somit besser.

Für das Vergleichsbeispiel 1 und das erfindungsgemäße Beispiel 1 werden für ein weiteres Szenario Simulationsrechnungen durchgeführt. Nach einer Simulationszeit von einer Stunde wird der Propenmassenstrom 121 zum Reaktor instantan um 10% herabgesenkt. Nach weiteren vier Stunden Simulationszeit wird der Propenmassenstrom wieder auf seinen ursprünglichen Wert zurückgesetzt. Hierbei wird für beide Beispiele auch kein zweiter Wärmeübertrager 142 hinzugeschaltet.

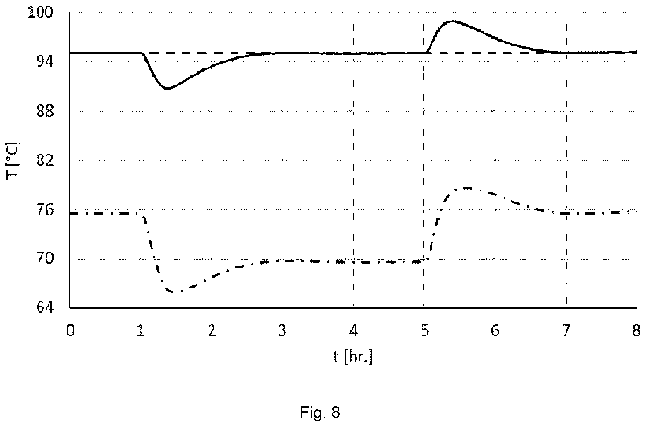

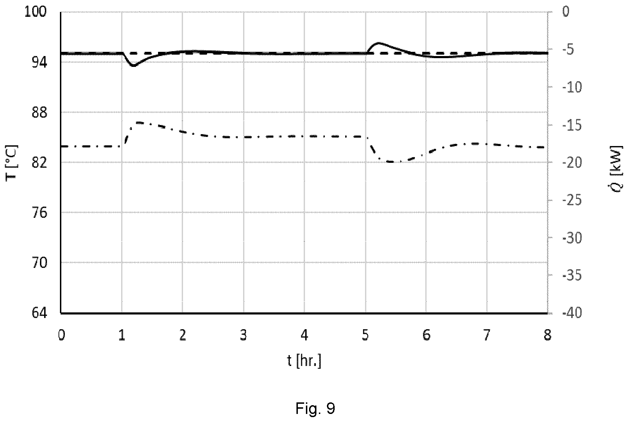

In Fig. 8 und Fig. 9 zeigt jeweils die gestrichelte Linie den Sollwert der Reaktortemperatur, die durchgezogene Linie den Istwert der Reaktortemperatur und die strich-gepunktete Linie das Stellsignal des Führungsreglers 105. Wie im Fall des Sollwertsprungs ist aus einem Vergleich der Kurven der Istwerte der Reaktortemperatur klar zu erkennen, dass durch das erfindungsgemäße Verfahren der Sollwert signifikant schneller erreicht wird als durch das Verfahren gemäß dem Stand der Technik. Die Regelgüte ist somit auch in diesem Szenario besser.

Für das Vergleichsbeispiel 1 und das erfindungsgemäße Beispiel 1 werden für ein weiteres Szenario folgende Simulationsrechnungen durchgeführt. Nach einer Stunde Simulationszeit wird ein zweiter Wärmeübertrager 142 zugeschaltet. Der Wärmestrom, der der Flüssigkeit im Primärkreislauf durch den zweiten Wärmeübertrager entzogen wird, wurde auf 10% des bis dahin vom ersten Wärmeübertrager 103 entzogenen Wärmestroms festgelegt. Die durch die Reaktion freigesetzte Wärmemenge bleibt davon unberührt. Nach fünf Stunden Simulationszeit wird der zweite Wärmeübertrager 142 wieder abgeschaltet und der erste Wärmeübertrager übernimmt wieder den gesamten Wärmestrom.

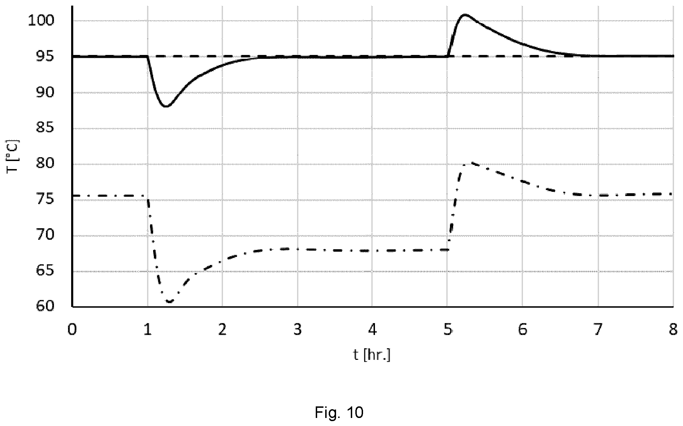

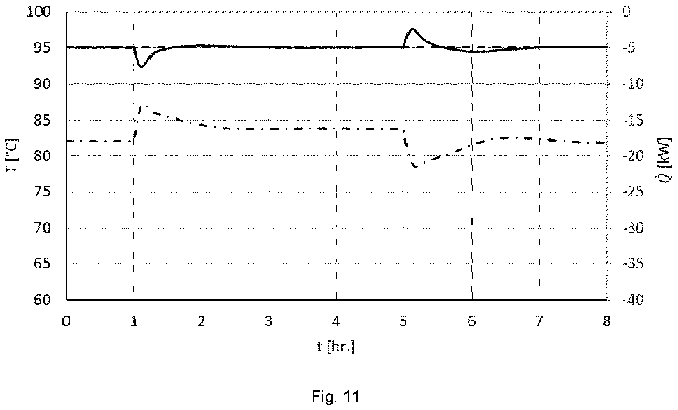

In Fig. 10 und Fig. 11 zeigt jeweils die gestrichelte Linie den Sollwert der Reaktortemperatur, die durchgezogene Linie den Istwert der Reaktortemperatur und die strich-gepunktete Linie das Stellsignal des Führungsreglers 105. Wie im Fall der vorherigen Simulationsbeispiele ist aus einem Vergleich der Kurven der Istwerte der Reaktortemperatur klar zu erkennen, dass durch das erfindungsgemäße Verfahren der Sollwert signifikant schneller erreicht wird als durch das Verfahren gemäß dem Stand der Technik. Die Regelgüte ist somit auch in diesem Szenario besser.

Diese Simulationsergebnisse zeigen, dass bei einer Verwendung einer Temperatur-Wärmestrom-Kaskade eine deutlich bessere Regelgüte als bei einer Verwendung einer Temperatur-Temperatur-Kaskade gemäß dem Stand der Technik erreicht wird.

**Patentansprüche**

1. Verfahren zur Regelung der Temperatur in einem verfahrenstechnischen Apparat (101, 201, 301, 401), bei dem in einem Primärkreislauf (102, 202, 302, 402) eine Flüssigkeit aus dem Apparat (101, 201, 301, 401) gefördert, zumindest teilweise einem Wärmeübertrager (103, 203, 303, 403) zugeführt und zumindest teilweise dem Apparat (101, 201, 301, 401) wieder zurückgeführt wird, wobei der Wärmeübertrager (103, 203, 303, 403) durch ein Wärmeträgermedium in einem Sekundärkreislauf (104, 204, 304, 404) gekühlt oder beheizt wird, umfassend die Schritte

   - Bereitstellen eines Sollwertes der Temperatur der Flüssigkeit im Apparat (101, 201, 301, 401),
   - Erfassen eines Istwertes der Temperatur der Flüssigkeit im Apparat (101, 201, 301, 401),
   - Berechnen der Temperaturdifferenz zwischen dem Istwert und dem Sollwert der Flüssigkeit im Apparat (101, 201, 301, 401),
   **dadurch gekennzeichnet, dass**
   - ein der Flüssigkeit im Primärkreislauf (102, 202, 302, 402) durch den Wärmeübertrager (103, 203, 303, 403) entnommener oder hinzugefügter Wärmestrom ermittelt wird,
   - auf Basis eines vorgegebenen Regelungsalgorithmus ein Stellsignal berechnet wird, wobei der Regelungsalgorithmus derart konfiguriert ist, dass das Stellsignal sich in Abhängigkeit von dem Wärmestrom und der Temperaturdifferenz zwischen dem Istwert und dem Sollwert der Flüssigkeit im Apparat (101, 201, 301, 401) ergibt, und
   - die Durchflussmenge des Stromes der Flüssigkeit durch den Wärmeübertrager (103, 203, 303, 403) im Primärkreislauf (102, 202, 302, 402) und/oder ein Mengenstrom des Wärmeträgermediums durch den Wärmeübertrager (103, 203, 303, 403) im Sekundärkreislauf (104, 204, 304, 404) auf Basis des Stellsignals manipuliert werden.

2. Verfahren nach Anspruch 1, **dadurch gekennzeichnet, dass** der Wärmestrom aus dem Flüssigkeitsstrom durch den Wärmeübertrager (103, 203, 303, 403) im Primärkreislauf (102, 202, 302, 402) sowie aus einer Temperaturdifferenz zwischen der Temperatur der Flüssigkeit vor und nach dem Wärmeübertrager (103, 203, 303, 403) im Primärkreislauf (102, 202, 302, 402) und/oder der Wärmestrom aus einem Mengenstrom des Wärmeträgermediums durch den Wärmeübertrager (103, 203, 303, 403) im Sekundärkreislauf (104, 204, 304, 404) sowie aus einer Temperaturdifferenz zwischen der Temperatur des Wärmeträgermediums vor und nach dem Wärmeübertrager (103, 203, 303, 403) im Sekundärkreislauf (104, 204, 304, 404) ermittelt wird.

3. Verfahren nach Anspruch 1 oder 2, **dadurch gekennzeichnet, dass** der Regelungsalgorithmus einen Temperaturregler und einen Wärmestromregler umfasst, wobei der Temperaturregler in Abhängigkeit von der Temperaturdifferenz zwischen dem Istwert und dem Sollwert der Flüssigkeit im Apparat (101, 201, 301, 401) als Ausgangssignal einen Sollwert für den der Flüssigkeit im Primärkreislauf (102, 202, 302, 402) durch den Wärmeübertrager (103, 203, 303, 403) entnommenen oder hinzugefügten Wärmestrom berechnet, dieser Sollwert an den Wärmestromregler übergeben wird, und der Wärmestromregler in Abhängigkeit von der Differenz zwischen dem Istwert und dem Sollwert des Wärmestroms als Ausgangssignal das Stellsignal zur Manipulation des Flüssigkeitsstroms durch den Wärmeübertrager (103, 203, 303, 403) im Primärkreislauf (102, 202, 302, 402) und/oder zur Manipulation des Mengenstroms des Wärmeträgermediums durch den Wärmeübertrager (103, 203, 303, 403) im Sekundärkreislauf (104, 204, 304, 404) generiert.

4. Verfahren nach einem der vorhergehenden Ansprüche, **dadurch gekennzeichnet, dass** im Primärkreislauf (102, 202, 302, 402) zumindest ein Bypass (307, 407) parallel zu dem Wärmeübertrager (103, 203, 303, 403) geschaltet ist.

5. Verfahren nach einem der vorhergehenden Ansprüche, **dadurch gekennzeichnet, dass** im Primärkreislauf (102, 202, 302, 402) in der Zuleitung zum Wärmeübertrager (103, 203, 303, 403) und/oder in der Ableitung vom Wärmeübertrager (103, 203, 303, 403) und/oder im Bypass (307, 407) mindestens ein Stellorgan zur Manipulation des Flüssigkeitsstroms auf Basis des Stellsignals vorhanden ist.

**6.** Verfahren nach einem der vorhergehenden Ansprüche, **dadurch gekennzeichnet, dass** im Sekundärkreislauf (104, 204, 304, 404) in der Zuleitung zum Wärmeübertrager (103, 203, 303, 403) und/oder in der Ableitung vom Wärmeübertrager (103, 203, 303, 403) mindestens ein Stellorgan zur Manipulation des Mengenstroms des Wärmeträgermediums auf Basis des Stellsignals vorhanden ist.

**7.** Verfahren nach einem der vorhergehenden Ansprüche, **dadurch gekennzeichnet, dass** der Regelungsalgorithmus eine Split-Range-Regelung umfasst, bei der anstatt eines Stellsignals mindestens zwei Stellsignale berechnet und mindestens zwei Ströme im Primärkreislauf und/oder im Sekundärkreislauf manipuliert werden, wobei ein erster Strom in Abhängigkeit von einem ersten Stellsignal und ein zweiter Strom in Abhängigkeit von einem zweiten Stellsignal manipuliert werden.

**8.** Verfahren nach einem der vorhergehenden Ansprüche, **dadurch gekennzeichnet, dass** der aus dem Apparat (101, 201, 301, 401) in den Primärkreislauf (102, 202, 302, 402) geförderte Flüssigkeitsstrom auf einen vorgegebenen Sollwert eingestellt wird.

**9.** Verfahren nach einem der vorhergehenden Ansprüche, **dadurch gekennzeichnet, dass** der Regelungsalgorithmus bei der Berechnung des Stellsignals mindestens eine Störgröße berücksichtigt, wobei die Störgröße eine gemessene oder geschätzte Prozessgröße umfasst, insbesondere mindestens einen Rohstoffstrom und/oder mindestens einen weiteren Wärmestrom.

**10.** Verfahren nach einem der vorhergehenden Ansprüche, **dadurch gekennzeichnet, dass** in dem Apparat (101, 201, 301, 401) eine chemische oder biologische, endotherme oder exotherme Reaktion oder ein exothermer oder endothermer physikalischer Prozess abläuft.

**11.** Verfahren nach Anspruch 10, **dadurch gekennzeichnet, dass** es sich bei der Reaktion um eine Hydroformylierung, Veretherung, Etherrückspaltung, Enalisierung, Dehydrierung, Pyrolyse, Hydrierung oder einen Crackprozess handelt.

**12.** Verfahren nach Anspruch 11, **dadurch gekennzeichnet, dass** die Hydrierung eine Vollhydrierung, Selektivhydrierung oder Kernhydrierung ist.

**Claims**

**1.** A method of closed-loop control of the temperature in a chemical engineering apparatus (101, 201, 301, 401), in which, in a primary circuit (102, 202, 302, 402), a liquid is conveyed out of the apparatus (101, 201, 301, 401), fed at least partly to a heat transferer (103, 203, 303, 403) and recycled at least partly back to the apparatus (101, 201, 301, 401), where the heat transferer (103, 203, 303, 403) is cooled or heated by a heat transfer medium in a secondary circuit (104, 204, 304, 404), comprising the steps of

- providing a target value for the temperature of the liquid in the apparatus (101, 201, 301, 401),
- detecting an actual value for the temperature of the liquid in the apparatus (101, 201, 301, 401),
- calculating the temperature difference between the actual value and the target value of the liquid in the apparatus (101, 201, 301, 401),
wherein
- a heat flow taken from or added to the liquid in the primary circuit (102, 202, 302, 402) by the heat transferer (103, 203, 303, 403) is ascertained,
- a control signal is calculated on the basis of a defined closed-loop control algorithm, where the closed-loop control algorithm is configured such that the control signal is dependent on the heat flow and the temperature difference between the actual value and the target value of the liquid in the apparatus (101, 201, 301, 401), and
- the flow rate of the stream of liquid through the heat transferer (103, 203, 303, 403) in the primary circuit (102, 202, 302, 402) and/or a flow rate of the heat transfer medium through the heat transferer (103, 203, 303, 403) in the secondary circuit (104, 204, 304, 404) is/are manipulated on the basis of the control signal.

**2.** The method according to claim 1, wherein the heat flow is ascertained from the liquid flow through the heat transferer (103, 203, 303, 403) in the primary circuit (102, 202, 302, 402) and from a temperature difference between the temperature of the liquid upstream and downstream of the heat transferer (103, 203, 303, 403) in the primary circuit (102, 202, 302, 402) and/or the heat flow is ascertained from a flow rate of the heat transfer medium through the heat transferer (103, 203, 303, 403) in the secondary circuit (104, 204, 304, 404) and from a temperature difference between the temperature of the heat transfer medium upstream and downstream of the heat transferer (103, 203, 303, 403) in the secondary circuit (104, 204, 304, 404).

3. The method according to claim 1 or 2, wherein the closed-loop control algorithm comprises a temperature controller and a heat flow controller, where the output signal calculated by the temperature controller is dependent on the temperature difference between the actual value and the target value of the liquid in the apparatus (101, 201, 301, 401) and is a target value for the heat flow taken from or added to the liquid in the primary circuit (102, 202, 302, 402) through the heat transferer (103, 203, 303, 403), this target value is transmitted to the heat flow controller, and the output signal generated by the heat flow controller is dependent on the difference between the actual value and the target value of the heat flow and is the control signal for manipulation of the liquid flow through the heat transferer (103, 203, 303, 403) in the primary circuit (102, 202, 302, 402) and/or for manipulation of the flow rate of the heat transfer medium through the heat transferer (103, 203, 303, 403) in the secondary circuit (104, 204, 304, 404).

4. The method according to any of the preceding claims, wherein there is at least one bypass (307, 407) in the primary circuit (102, 202, 302, 402) that runs parallel to the heat transferer (103, 203, 303, 403).

5. The method according to any of the preceding claims, wherein there is at least one control unit for manipulation of the liquid flow on the basis of the control signal in the primary circuit (102, 202, 302, 402) in the feed to the heat transferer (103, 203, 303, 403) and/or in the drain from the heat transferer (103, 203, 303, 403) and/or in the bypass (307, 407).

6. The method according to any of the preceding claims, **characterized in that** there is at least one control unit for manipulation of the flow rate of the heat transfer medium on the basis of the control signal in the secondary circuit (104, 204, 304, 404) in the feed to the heat transferer (103, 203, 303, 403) and/or in the drain from the heat transferer (103, 203, 303, 403).

7. The method according to any of the preceding claims, wherein the closed-loop control algorithm has a split range control function where, rather than one control signal, at least two control signals are calculated and at least two streams in the primary circuit and/or in the secondary circuit are manipulated, where a first stream is manipulated as a function of a first control signal and a second stream as a function of a second control signal.

8. The method according to any of the preceding claims, **characterized in that** the liquid flow conveyed from the apparatus (101, 201, 301, 401) into the primary circuit (102, 202, 302, 402) is set to a defined target value.

9. The method according to any of the preceding claims, **characterized in that** the closed-loop control algorithm, in calculating the control signal, takes account of at least one perturbation parameter, where the perturbation parameter comprises a measured or estimated process parameter, especially at least one raw material flow and/or at least one further heat flow.

10. The method according to any of the preceding claims, **characterized in that** a chemical or biological, endothermic or exothermic reaction or an exothermic or endothermic physical process is proceeding in the apparatus (101, 201, 301, 401).

11. The method according to claim 10, wherein the reaction is a hydroformylation, etherification, ether cleavage, enalization, dehydrogenation, pyrolysis, hydrogenation, or a cracking process.

12. The method according to claim 11, wherein the hydrogenation is a full hydrogenation, selective hydrogenation or ring hydrogenation.

**Revendications**

1. Procédé de régulation en boucle fermée de la température dans un appareil d'ingénierie de processus (101, 201, 301, 401), dans lequel, dans un circuit primaire (102, 202, 302, 402), un liquide est transporté hors de l'appareil (101, 201, 301, 401), est au moins en partie délivré à un échangeur de chaleur (103, 203, 303, 403) et est au moins en partie renvoyé à l'appareil (101, 201, 301, 401), l'échangeur de chaleur (103, 203, 303, 403) étant refroidi ou chauffé par un milieu caloporteur dans un circuit secondaire (104, 204, 304, 404), comprenant les étapes suivantes

- fournir une valeur de consigne de la température du liquide dans l'appareil (101, 201, 301, 401),
- détecter une valeur réelle de la température du liquide dans l'appareil (101, 201, 301, 401),
- calculer la différence de température entre la valeur réelle et la valeur de consigne du liquide dans l'appareil (101, 201, 301, 401),

**caractérisé en ce que**

- un flux de chaleur prélevé ou ajouté au liquide dans le circuit primaire (102, 202, 302, 402) par l'échangeur de chaleur (103, 203, 303, 403) est déterminé,
- un signal de réglage est calculé sur la base d'un algorithme de régulation en boucle fermée prédéterminé, l'algorithme de régulation en boucle fermée étant configuré de telle sorte que le signal de réglage soit obtenu en fonction du flux de chaleur et de la différence de température entre la valeur réelle et la valeur de consigne du liquide dans l'appareil (101, 201, 301, 401), et
- le débit de l'écoulement du liquide à travers l'échangeur de chaleur (103, 203, 303, 403) dans le circuit primaire (102, 202, 302, 402) et/ou un débit massique du milieu caloporteur à travers l'échangeur de chaleur (103, 203, 303, 403) dans le circuit secondaire (104, 204, 304, 404) sont manipulés sur la base du signal de réglage.

2. Procédé selon la revendication 1, **caractérisé en ce que** le flux de chaleur est déterminé à partir de l'écoulement de liquide à travers l'échangeur de chaleur (103, 203, 303, 403) dans le circuit primaire (102, 202, 302, 402) ainsi qu'à partir d'une différence de température entre la température du liquide avant et après l'échangeur de chaleur (103, 203, 303, 403) dans le circuit primaire (102, 202, 302, 402) et/ou **en ce que** le flux de chaleur est déterminé à partir d'un débit massique du milieu caloporteur à travers l'échangeur de chaleur (103, 203, 303, 403) dans le circuit secondaire (104, 204, 304, 404) ainsi qu'à partir d'une différence de température entre la température du milieu caloporteur avant et après l'échangeur de chaleur (103, 203, 303, 403) dans le circuit secondaire (104, 204, 304, 404).

3. Procédé selon la revendication 1 ou 2, **caractérisé en ce que** l'algorithme de régulation en boucle fermée comprend un régulateur de température et un régulateur de flux de chaleur, le régulateur de température calculant en tant que signal de sortie, en fonction de la différence de température entre la valeur réelle et la valeur de consigne du liquide dans l'appareil (101, 201, 301, 401), une valeur de consigne du flux de chaleur prélevé ou ajouté au liquide dans le circuit primaire (102, 202, 302, 402) par l'échangeur de chaleur (103, 203, 303, 403), ladite valeur de consigne étant transmise au régulateur de flux de chaleur, et le régulateur de flux de chaleur générant en tant que signal de sortie, en fonction de la différence entre la valeur réelle et la valeur de consigne du flux de chaleur, le signal de réglage destiné à manipuler l'écoulement de liquide à travers l'échangeur de chaleur (103, 203, 303, 403) dans le circuit primaire (102, 202, 302, 402) et/ou à manipuler le débit massique du milieu caloporteur à travers l'échangeur de chaleur (103, 203, 303, 403) dans le circuit secondaire (104, 204, 304, 404).

4. Procédé selon l'une des revendications précédentes, **caractérisé en ce qu'**au moins une dérivation (307, 407) est montée en parallèle avec l'échangeur de chaleur (103, 203, 303, 403) dans le circuit primaire (102, 202, 302, 402) .

5. Procédé selon l'une des revendications précédentes, **caractérisé en ce qu'**au moins un organe de réglage est présent dans le circuit primaire (102, 202, 302, 402) dans la ligne d'alimentation de l'échangeur de chaleur (103, 203, 303, 403) et/ou dans la ligne de sortie de l'échangeur de chaleur (103, 203, 303, 403) et/ou dans la dérivation (307, 407) pour manipuler l'écoulement de liquide sur la base du signal de réglage.

6. Procédé selon l'une des revendications précédentes, **caractérisé en ce qu'**au moins un organe de réglage est présent dans le circuit secondaire (104, 204, 304, 404) dans la ligne d'alimentation de l'échangeur de chaleur (103, 203, 303, 403) et/ou dans la ligne de sortie de l'échangeur de chaleur (103, 203, 303, 403) pour manipuler le débit massique du milieu caloporteur sur la base du signal de réglage.

7. Procédé selon l'une des revendications précédentes, **caractérisé en ce que** l'algorithme de régulation en boucle fermée comprend une régulation à plage fractionnée conformément à laquelle, au lieu d'un signal de réglage, au moins deux signaux de réglage sont calculés et au moins deux débits sont manipulés dans le circuit primaire et/ou dans le circuit secondaire, un premier débit étant manipulé en fonction d'un premier signal de réglage et un deuxième débit en fonction d'un second signal de réglage.

8. Procédé selon l'une des revendications précédentes, **caractérisé en ce que** l'écoulement de liquide transporté par l'appareil (101, 201, 301, 401) dans le circuit primaire (102, 202, 302, 402) est réglé à une valeur de consigne prédéterminée.

**9.** Procédé selon l'une des revendications précédentes, **caractérisé en ce que** l'algorithme de régulation en boucle fermée prend en compte au moins une grandeur perturbatrice dans le calcul du signal de réglage, ladite grandeur perturbatrice comprenant une grandeur de processus mesurée ou estimée, notamment au moins un écoulement de matière première et/ou au moins un autre flux de chaleur.

**10.** Procédé selon l'une des revendications précédentes, **caractérisé en ce qu'**une réaction chimique ou biologique, endothermique ou exothermique, ou un processus physique exothermique ou endothermique, se déroule dans l'appareil (101, 201, 301, 401).

**11.** Procédé selon la revendication 10, **caractérisé en ce que** la réaction est une hydroformylation, une éthérification, une décomposition d'éther, une énolisation, une déshydrogénation, une pyrolyse, une hydrogénation ou un processus de craquage.

**12.** Procédé selon la revendication 11, **caractérisé en ce que** l'hydrogénation est une hydrogénation totale, une hydrogénation sélective ou une hydrogénation de noyau.

Fig. 1

Fig. 2

Fig. 3

EP 4 323 101 B1

Fig. 4

Fig. 5

Fig. 6

Fig. 7

Fig. 8

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015047723 A1

**In der Beschreibung aufgeführte Nicht-Patentliteratur**

- **H. SCHULER.** Prozessführung. Oldenbourg Verlag, 1999, 165-171
- Process Control and Optimization. **B. G. LIPTAK.** Instrument Engineers' Handbook. Taylor & Francis Inc, 2005, vol. Two
- VDI-Wärmeatlas. Springer Vieweg, 2019
- **OTTO FÖLLINGER.** Regelungstechnik. Hüthig Verlag, 1994
- **HEINZ UNBEHAUEN.** Regelungstechnik I. Vieweg Verlag, 2005
- **S. SKOGESTAD ; LAN POSTLETHWAITE.** Multivariable Feedback Control. Wiley Verlag, 2005